(19) Europäisches Patentamt

European Patent Office

Office européen des brevets

(11) **EP 2 886 193 A2**

(12) **EUROPEAN PATENT APPLICATION**

(43) Date of publication:
24.06.2015 Bulletin 2015/26

(51) Int Cl.:
*B01J 27/16* (2006.01)  *C07C 45/29* (2006.01)
*C07C 47/22* (2006.01)  *C07B 61/00* (2006.01)

(21) Application number: 14004139.3

(22) Date of filing: 28.03.2011

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 31.03.2010 JP 2010084563
31.03.2010 JP 2010084562
22.09.2010 JP 2010212528
22.09.2010 JP 2010212527
30.09.2010 JP 2010222802
30.09.2010 JP 2010222801

(62) Document number(s) of the earlier application(s) in
accordance with Art. 76 EPC:
11765523.3 / 2 554 258

(71) Applicant: NIPPON SHOKUBAI CO., LTD.
Osaka-shi, Osaka-fu 541 (JP)

(72) Inventors:
• Ezawa, Takayuki
Tsukuba-shi, Ibaraki, 3050856 (JP)
• Okada, Masaki
Tsukuba-shi, Ibaraki, 3050856 (JP)
• Arita, Yoshitaka
Tsukuba-shi, Ibaraki, 3050856 (JP)

(74) Representative: Müller-Boré & Partner
Patentanwälte PartG mbB
Friedenheimer Brücke 21
80639 München (DE)

Remarks:
This application was filed on 08.12.2014 as a
divisional application to the application mentioned
under INID code 62.

(54) **Catalyst for glycerin dehydration, and process for producing acrolein, process for producing acrylic acid, and process for producing hydrophilic resin each using the catalyst**

(57) A catalyst for glycerin dehydration of the present invention comprises a rare-earth metal phosphate, wherein a molar ratio P/R of phosphorus (P) to a rare-earth metal (R) is more than 1.0 and 2.0 or less. An another catalyst for glycerin dehydration of the present invention comprises a combination of a rare-earth metal phosphate and a metal element other than a rare-earth metal, wherein a molar ratio M/(P+R) of a metal element (M) to phosphorus (P) and a rare-earth metal (R) is more than 0.00005 and 0.5 or less.

EP 2 886 193 A2

**Description**

TECHNICAL FIELD

The present invention relates to a catalyst used for dehydration reaction of glycerin, and process for producing acrolein, process for producing acrylic acid, and process for producing a hydrophilic resin such as a water-absorbent resin and a water-soluble resin, each using the catalyst.

BACKGROUND ART

Biodiesel fuels produced from vegetable oils have drawn much attention as alternate fuels for fossil fuels and also in terms of low emission of carbon dioxide, and therefore, an increase in demand for them has been expected. Since the production of such biodiesel fuels is accompanied by formation of glycerin as a by-product, it is required to make effective utilization of glycerin.

As an example of effective utilization of glycerin, a process for producing acrolein from glycerin as a raw material is known. For example, Patent Literatures 1 to 3 disclose glycerin dehydration catalysts comprising boron phosphate and processes for producing acrolein using the catalyst. In addition, Patent Literature 4 discloses a glycerin dehydration catalyst comprising a rare-earth metal phosphate and a process for producing acrolein using the catalyst.

CITATION LIST

PATENT LITERATURE

PATENT LITERATURE 1
International Publication WO 2007/119528
PATENT LITERATURE 2
Japanese Unexamined Patent Application Publication No. 2008-307521
PATENT LITERATURE 3
Japanese Unexamined Patent Application Publication No. 2009-263284
PATENT LITERATURE 4
Japanese Unexamined Patent Application Publication No. 2009-274982

SUMMARY OF INVENTION

TECHNICAL PROBLEM

The glycerin dehydration catalysts disclosed in Patent Literatures 1 to 3 generally comprises boron phosphate ($BPO_4$) whose molar ratio P/B of phosphorus (P) to boron (B) is 1.0. These glycerin dehydration catalysts demonstrate relatively high acrolein selectivity; however, there were problems that propionaldehyde was produced as a by-product, or the like. In the catalyst disclosed in Patent Literature 4, Y, La, Ce, Pr or Nd is used as a rare-earth metal, and the catalyst is prepared by blending raw materials so that the molar ratio P/R of phosphorus (P) to the rare-earth metal (R) is 1.0. The glycerin dehydration catalyst disclosed in Patent Literature 4 also demonstrates relatively-high acrolein selectivity; however, there were problems that propionaldehyde was produced as a by-product. Acrolein can be used for a raw material of a hydrophilic resin such as polyacrylic acid, and when acrolein contains propionaldehyde in a large amount, polyacrylic acid (a hydrophilic resin) produced from the acrolein via acrylic acid comes to contain propionic acid derived from propionaldehyde, that causes odor and is unfavorable.

The present invention has been achieved in view of the above circumstances, and the object of the present invention is to provide a catalyst for glycerin dehydration that is able to reduce the production of propionaldehyde, a by-product, and produce acrolein in high yield, and a process for producing acrolein, a process for producing acrylic acid, and a process for producing a hydrophilic resin, each using the catalyst.

SOLUTION TO PROBLEM

A catalyst for glycerin dehydration of the present invention comprises boron phosphate or a rare-earth metal phosphate, wherein a molar ratio P/B of phosphorus (P) to boron (B) or a molar ratio P/R of phosphorus (P) to a rare-earth metal (R) is more than 1.0 and 2.0 or less. The present invention also provides a catalyst for glycerin dehydration

comprising a combination of boron phosphate and a metal element or a combination of a rare-earth metal phosphate and a metal element other than a rare-earth metal, wherein a molar ratio M/(P+B) of a metal element (M) to phosphorus (P) and boron (B) or a molar ratio M/(P+R) of a metal element (M) to phosphorus (P) and a rare-earth metal (R) is more than 0.00005 and 0.5 or less. By using the catalyst of the present invention, selectivity of propionaldehyde, a by-product, can be reduced while yield of acrolein is not severely decreased in dehydration reaction of glycerin. Further, performance of the catalyst can be maintained at a high level for a long period depending on conditions.

The present invention also provides a process for producing acrolein comprising the step of dehydrating glycerin in the presence of the catalyst of the present invention to produce acrolein. Preferably, in this producing process, glycerin is dehydrated by gas-phase reaction of bringing a reaction gas containing glycerin gas into contact with the catalyst.

The present invention further provides a process for producing acrylic acid comprising the step of oxidizing acrolein produced by the above acrolein producing process to produce acrylic acid, and a process for producing a hydrophilic resin comprising the step of polymerizing a monomeric component including the thus obtained acrylic acid. The hydrophilic resin obtained by the producing process of the present invention contains small amounts of propionic acid, which is derived from propionaldehyde and causes odor; and therefore, the hydrophilic resin can be suitably used for a water-absorbent resin and the like, that is utilized for a disposable diaper and the like.

ADVANTAGEOUS EFFECTS OF INVENTION

According to the catalyst for glycerin dehydration of the present invention, production of propionaldehyde can be reduced while yield of acrolein is not severely decreased. When using this catalyst, acrolein can be produced in high yield accompanied by lesser amount of propionaldehyde of a by-product in dehydration reaction of glycerin. When the thus obtained acrolein is used, a water-absorbent resin which gives out a very little odor can be easily produced via acrylic acid, for example.

BRIEF DESCRIPTION OF DRAWINGS

Fig. 1 shows a graph indicating a result of Example of the present invention.
Fig. 2 shows a graph indicating a result of Example of the present invention.

DESCRIPTION OF THE PREFERRED EMBODIMENTS

[Glycerin dehydration catalyst]

Firstly, a catalyst for glycerin dehydration according to the present first invention is explained. A catalyst for glycerin dehydration of the present first invention comprises boron phosphate or a rare-earth metal phosphate, wherein a molar ratio P/B of phosphorus (P) to boron (B) or a molar ratio P/R of phosphorus (P) to a rare-earth metal (R) is more than 1.0 and not more than 2.0. The molar ratio P/B and the molar ratio P/R are respectively calculated from blending amounts of raw materials used in the preparation of the boron phosphate or the rare-earth metal phosphate. In the case where the catalyst comprising a rare-earth metal phosphate contains two or more kinds of elements as the rare-earth metal (R), the R of the molar ratio R/P means the sum of quantities of two or more kinds of the rare-earth metal elements. In the present invention, a "rare-earth metal" means a "rare-earth metal element".

In the catalyst for glycerin dehydration comprising boron phosphate or a rare-earth metal phosphate, the molar ratio P/B or the molar ratio P/R is generally more than 1.0, preferably 1.02 or more, more preferably 1.05 or more, and generally 2.0 or less, preferably 1.5 or less, more preferably less than 1.5, even more preferably 1.4 or less, and particularly preferably 1.3 or less. When the molar ratio P/B or the molar ratio P/R is adjusted in the proper range, selectivity of propionaldehyde (PALD; propanal), a by-product, can be reduced while yield of acrolein formed in dehydration reaction of glycerin is not severely decreased. Acrolein is used for a raw material of polyacrylic acid that is available for a water-absorbent resin or the like, for example. In such case, when acrolein contains a large amount of propionaldehyde, polyacrylic acid produced from the acrolein via acrylic acid comes to contain propionic acid derived from propionaldehyde, that causes odor and is unfavorable. In addition, when the molar ratio P/B or the molar ratio P/R is adjusted in the proper range, performance of the catalyst is easily maintained at a high level for a long period, or increase of pressure loss, due to the deposition of carbonaceous matters and the like, is suppressed during the reaction, resulting in easily conducting the reaction for a long period. Particularly, in the catalyst for glycerin dehydration comprising a rare-earth metal phosphate, the molar ratio P/R is preferably less than 1.5 in view of maintaining the catalyst performance at a high level for a long period.

In the catalyst of the first invention, the form of the phosphate which constitutes the boron phosphate or the rare-earth metal phosphate is not particularly limited. Examples of the phosphate constituting these phosphates include orthophosphate; condensed phosphate such as pyrophosphate, triphosphate, polyphosphate, metaphosphate and ultraphosphate; and the like. These phosphates may be contained either one kind or at least two kinds of them.

In the catalyst of the first invention, the rare-earth metal element which constitutes the rare-earth metal phosphate may be any element of scandium (Sc), yttrium (Y), lanthanum (La), cerium (Ce), praseodymium (Pr), neodymium (Nd), promethium (Pm), samarium (Sm), europium (Eu), gadolinium (Gd), terbium (Tb), dysprosium (Dy), holmium (Ho), erbium (Er), thulium (Tm), ytterbium (Yb) and lutetium (Lu). These rare-earth metal elements may be contained either one kind or at least two kinds of them. However, promethium (Pm) is not preferably used since it is radioactive. Among these rare-earth metal elements, in view of demonstrating superior catalyst performance, at least one element selected from the group consisting of yttrium, lanthanum, cerium, praseodymium, neodymium and gadolinium is preferable, at least one element selected from the group consisting of yttrium, cerium and neodymium is more preferable, and neodymium is even more preferable.

In the catalyst of the first invention, the catalyst for glycerin dehydration comprising boron phosphate may contain other component, as long as the molar ratio P/B of phosphorus (P) to boron (B) is in the prescribed range. The boron phosphate contained in the catalyst may be either only one kind or at least two kinds of them, as long as the molar ratio P/B is in the prescribed range.

In the catalyst of the first invention, the catalyst for glycerin dehydration comprising a rare-earth metal phosphate may contain other component, as long as the molar ratio P/R of phosphorus (P) to the rare-earth metal (R) is in the prescribed range. The rare-earth metal phosphate contained in the catalyst may be either only one kind or at least two kinds of them, as long as the molar ratio P/R is in the prescribed range.

As the catalyst for glycerin dehydration comprising a rare-earth metal phosphate, the catalyst containing neodymium, yttrium or cerium as the rare-earth metal is also preferred. That is, it is also preferred that the catalyst comprises neodymium phosphate, yttrium phosphate or cerium phosphate, wherein a molar ratio P/Nd of phosphorus (P) to neodymium (Nd), a molar ratio P/Y of phosphorus (P) to yttrium (Y), or a molar ratio P/Ce of phosphorus (P) to cerium (Ce) is in the prescribed range. When neodymium, yttrium or cerium is employed as the rare-earth metal, a catalyst for glycerin dehydration which demonstrates high acrolein yield and low propionaldehyde selectivity is easily obtained.

Secondly, a catalyst for glycerin dehydration of the present second invention is explained. A catalyst for glycerin dehydration of the present second invention comprises a combination of boron phosphate and a metal element or a combination of a rare-earth metal phosphate and a metal element other than a rare-earth metal, wherein a molar ratio M/(P+B) of a metal element (M) to phosphorus (P) and boron (B) or a molar ratio M/(P+R) of a metal element (M) to phosphorus (P) and a rare-earth metal (R) is more than 0.00005 and not more than 0.5. The molar ratio M/(P+B) and the molar ratio M/(P+R) are respectively calculated from blending amounts of raw materials used in the preparation of the catalyst comprising boron phosphate and a metal element or the catalyst comprising a rare-earth metal phosphate and a metal element. In the case where the catalyst contains two or more kinds of elements as the rare-earth metal (R) or the metal element (M), the R and the M of the molar ratios M/(P+B) and M/(P+R) mean the sum of quantities of two or more kinds of the rare-earth metal elements and the sum of quantities of two or more kinds of the metal elements, respectively.

In the catalyst for glycerin dehydration comprising the combination of boron phosphate and a metal element or the combination of a rare-earth metal phosphate and a metal element, the molar ratio M/(P+B) or the molar ratio M/(P+R) is generally more than 0.00005, preferably 0.00009 or more, more preferably 0.0001 or more, even more preferably 0.0005 or more, and particularly preferably 0.001 or more, and generally 0.5 or less, preferably less than 0.5, more preferably 0.45 or less, even more preferably 0.4 or less, even more preferably 0.35 or less, and particularly preferably 0.25 or less. When the catalyst having the molar ratio M/(P+B) or the molar ratio M/(P+R) more than 0.00005 is used for the dehydration of glycerin, acrolein formed in the dehydration reaction of glycerin can be obtained in high yield, and selectivity of propionaldehyde (PALD; propanal), a by-product, can be reduced. Meanwhile, when the molar ratio M/(P+B) or the molar ratio M/(P+R) is excessively high, the effect of the addition of the metal element peaks out, and further, when it is much higher, the selectivity of acrolein may decrease and/or the selectivity of propionaldehyde may increase. In addition, a compound containing the metal element, that may be hereinafter referred to as a "metal source compound", comes to be consumed beyond necessity, the production cost is likely to rise.

In the catalyst of the second invention, with regard to the catalyst for glycerin dehydration comprising boron phosphate and a metal element, respective contents of phosphorus (P), boron (B) and the metal element (M) are not particularly limited, as long as the molar ratio M/(P+B) is in the prescribed range; however, it is preferred, for example, that a molar ratio P/B of phosphorus (P) to boron (B) and a molar ratio M/B of the metal element (M) to boron (B) are respectively within the following ranges. The molar ratio P/B is preferably 0.8 or more and 3.0 or less, more preferably 2.0 or less, even more preferably 1.5 or less, and particularly preferably 1.3 or less. The molar ratio of M/B is preferably 0.001 or more, more preferably 0.01 or more, and preferably 2.0 or less, more preferably 1.5 or less, even more preferably 1.0 or less, and particularly preferably 0.8 or less.

In the catalyst of the second invention, with regard to the catalyst for glycerin dehydration comprising a rare-earth metal phosphate and a metal element, respective contents of phosphorus (P), the rare-earth metal (R) and the metal element (M) are not particularly limited, as long as the molar ratio M/(P+R) is in the prescribed range; however, it is preferred, for example, that a molar ratio P/R of phosphorus (P) to the rare-earth metal (R) and a molar ratio M/R of the metal element (M) to the rare-earth metal (R) are respectively within the following ranges. The molar ratio P/R is preferably 0.7 or more and 2.0 or less, more preferably 1.8 or less, and even more preferably 1.6 or less. The molar ratio of M/R is preferably more than 0.0001, more preferably 0.001 or more, and preferably 1.5 or less, more preferably 1.0 or less, even more preferably 0.5 or less, and particularly preferably 0.3 or less.

In the catalyst of the second invention, the form of the phosphate which constitutes the boron phosphate or the rare-earth metal phosphate is not particularly limited; and the phosphate which constitutes the boron phosphate or the rare-earth metal phosphate in the catalyst of the first invention can be used. Further, in the catalyst of the second invention, as the rare-earth metal element which constitutes the rare-earth metal phosphate, the rare-earth metal which constitutes the rare-earth metal phosphate in the catalyst of the first invention can be used. The rare-earth metal element which constitutes the rare-earth metal phosphate contained in the catalyst of the second invention is preferably at least one element selected from the group constituting of yttrium, lanthanum, cerium, praseodymium, neodymium and gadolinium, and more preferably at least one element selected from the group consisting of yttrium, cerium and neodymium.

The metal element contained in the catalyst for glycerin dehydration comprising boron phosphate and a metal element is not particularly limited, as long as it is a metal element other than boron and exerts the effect of the present invention. The metal element is preferably at least one element selected from the group consisting of, for example, alkali metal elements, alkaline-earth metal elements, rare-earth metal elements, iron group elements, platinum group elements, copper group elements and aluminum group elements.

The metal element contained in the catalyst for glycerin dehydration comprising a rare-earth metal phosphate and a metal element is not particularly limited, as long as it is a metal element other than a rare-earth metal and exerts the effect of the present invention. The metal element is preferably at least one element selected from the group consisting of, for example, alkali metal elements, alkaline-earth metal elements, iron group elements, platinum group elements, copper group elements and aluminum group elements.

Examples of the alkali metal element include, for example, lithium, sodium, potassium, rubidium and cesium. Examples of the alkaline-earth metal element include, for example, beryllium, magnesium, calcium, strontium and barium. Examples of the rare-earth metal element include, for example, scandium, yttrium and lanthanoid such as lanthanum, cerium, praseodymium, neodymium, samarium, europium, gadolinium, terbium, dysprosium, erbium and ytterbium. Examples of the iron group element include iron, cobalt and nickel. Examples of the platinum group element include, for example, ruthenium, rhodium, palladium, iridium and platinum. Examples of the copper group element include copper, silver and gold. Examples of the aluminum group element include aluminum, gallium, indium and thallium. These metal elements may be contained either one kind or at least two kinds of them.

The metal element contained in the catalyst for glycerin dehydration comprising boron phosphate and a metal element is preferably at least one element selected from the group consisting of alkali metal elements, alkaline-earth metal elements, rare-earth metal elements, copper group elements and aluminum group elements, and more preferably at least one element selected from the group consisting of lithium, sodium, potassium, rubidium, cesium, magnesium, calcium, barium, cerium, silver and aluminum.

The metal element contained in the catalyst for glycerin dehydration comprising a rare-earth metal phosphate and a metal element is preferably at least one element selected from the group consisting of alkali metal elements, alkaline-earth metal elements and platinum group elements.

In the case where two or more kinds of the metal elements are used in combination, the M of the molar ratios M/(P+B), M/(P+R), M/B and M/R means the sum of molar quantities (unit: mol) of respective metal elements. That is, the M is calculated by the equation of $M=M_1+M_2+M_3...$, provided that the molar quantities of the metal elements are respectively labeled as $M_1$, $M_2$, $M_3$ and more. The same is applied to the rare-earth metal element when two or more kinds of the rare-earth metal elements are used in combination.

In the catalyst of the second invention, the catalyst for glycerin dehydration comprising boron phosphate and a metal element may contain an other component, as long as the molar ratio M/(P+B) of the metal element (M) to phosphorus (P) and boron (B) is in the prescribed range. The boron phosphate contained in the catalyst may be either only one kind or at least two kinds of them, as long as the molar ratio M/(P+B) is in the prescribed range.

In the catalyst of the second invention, the catalyst for glycerin dehydration comprising a rare-earth metal phosphate and a metal element other than a rare-earth metal may contain an other component, as long as the molar ratio M/(P+R) of the metal element (M) to phosphorus (P) and the rare-earth metal (R) is in the prescribed range. The rare-earth metal phosphate contained in the catalyst may be either only one kind or at least two kinds of them, as long as the molar ratio M/(P+R) is in the prescribed range. In view of demonstrating superior catalyst performance, the catalyst preferably contains yttrium phosphate, cerium phosphate or neodymium phosphate.

Preferably, the catalyst for glycerin dehydration comprising a rare-earth metal phosphate and a metal element contains a salt of phosphoric acid and at least one kind of the rare-earth metal selected from the group consisting of yttrium, lanthanum, cerium, praseodymium, neodymium and gadolinium, as the rare-earth metal phosphate, wherein the molar ratio M/(P+R) of the metal element (M) to phosphorus (P) and the rare-earth metal (R: Y, La, Ce, Pr, Nd, Gd) is in the prescribed range. It is also preferable that the catalyst for glycerin dehydration comprising a rare-earth metal phosphate and a metal element contains at least one element selected from the group consisting of alkali metal elements, alkaline-earth metal elements and platinum group elements as the metal element, wherein the molar ratio M/(P+R) of the metal element (M: alkali metal elements, alkaline-earth metal elements and platinum group elements) to phosphorus (P) and the rare-earth metal element (R) is in the prescribed range. Further, it is also preferable that the catalyst of the present invention is formed by the combination of them. By appropriately selecting the rare-earth metal and the metal element in these manners, a catalyst for glycerin dehydration demonstrating high acrolein yield and low propionaldehyde selectivity is easily obtained.

The boron phosphate or the rare-earth metal phosphate contained in the catalyst of the present invention is preferably has a crystalline structure in view of enhancing the catalyst performance. The crystalline structures of the boron phosphate and the rare-earth metal phosphate are not particularly limited, and may be, for example, tetragonal (for example, cristobalite), monoclinic, hexagonal, or the like. Whether the boron phosphate or the rare-earth metal phosphate contained in the catalyst has a crystalline structure can be confirmed by X-ray diffraction measurement of the catalyst. Here, the "catalyst of the present invention" includes the catalyst of the present first invention and the catalyst of the present second invention.

The catalyst of the present invention may be a supported catalyst in which the boron phosphate or the rare-earth metal phosphate is supported on a carrier. In the catalyst of the second invention, the metal element may be further supported on the carrier. Examples of the usable carrier include, for example, inorganic oxides such as silica, alumina, titania and zirconia, and complex oxides of them; crystalline metallosilicates such as zeolites; metals such as stainless steels and aluminum, and composition metals thereof; inorganic compounds such as activated carbon and silicon carbide; and the like.

The shape of the catalyst is not particularly limited, and may be, for example, spherical, column-shape, ring-shape, saddle-shape, honeycomb, or sponge-shape.

The catalyst of the first invention only has to comprise boron phosphate or a rare-earth metal phosphate as an active component. The catalyst of the second invention only has to comprise the combination of boron phosphate and a metal element or the combination of a rare-earth metal phosphate and a metal element as an active component. The catalyst which contains a larger amount of the active component is suitable for an industrial production of acrolein; and therefore, in the case where the catalyst of the present invention is used as a non-supported catalyst, the content of the active component is preferably 5 mass% or more, more preferably 20 mass% or more, even more preferably 40 mass% or more, and preferably 100 mass% or less, relative to 100 mass% of the catalyst. In the case where the catalyst of the present invention is used as a supported catalyst, the content of the active component is preferably 0.01 mass% or more, more preferably 1 mass% or more, even more preferably 5 mass% or more, and preferably 70 mass% or less, more preferably 60 mass% or less, even more preferably 50 mass% or less, relative to 100 mass% of the catalyst.

Since the catalyst of the present invention has the above-described constitution, selectivity of propionaldehyde of a by-product can be reduced while yield of acrolein is not severely decreased in dehydration reaction of glycerin. Further, the catalyst performance can be maintained at a high level for a long period depending on conditions.

[Preparation of glycerin dehydration catalyst]

The catalyst of the present invention can be prepared by a conventionally-known catalyst preparation method such as a kneading method, a condensation method, a precipitation method, a co-precipitation method, a sol-gel method, or a hydrothermal method. In a kneading method, operations of calcinating a solid matter (catalyst precursor) obtained by kneading raw compounds may be employed. In a condensation method, a precipitation method, a co-precipitation method, a sol-gel method, and a hydrothermal method, operations of calcinating a solid matter (catalyst precursor) obtained by adding raw compounds into a solvent and physically-treating according to the method may be employed. For example, in the condensation method, the solid matter (catalyst precursor) obtained by adding raw compounds into a solvent and dewatering to concentrate may be calcined. In the case of preparing the catalyst of the second invention by the condensation method, precipitation method, co-precipitation method, sol-gel method, or hydrothermal method, operations of adding a metal source compound to a calcinated product, which has been prepared by calcinating a solid matter obtained by adding raw compounds including a boron compound or a rare-earth metal compound in addition to a phosphate compound into a solvent and physically-treating according to the method, to give a catalyst precursor, and re-calcinating the catalyst precursor may be employed.

The raw compounds are appropriately adopted according to the kind of the catalyst. In the case of preparing the catalyst containing boron phosphate, raw compounds including a phosphate compound and a boron compound may be used. In the case of preparing the catalyst containing the rare-earth metal phosphate, raw compounds including a phosphate compound and a rare-earth metal compound may be used. In the case of preparing the catalyst containing boron phosphate and the metal element, raw compounds including a phosphate compound, a boron compound and a metal source compound may be used. In the case of preparing the catalyst containing the rare-earth metal phosphate and the metal element, raw compounds including a phosphate compound, a rare-earth metal compound and a metal source compound may be used.

The used amounts (blended amounts) of the phosphate compound, the boron compound, the rare-earth metal compound and the metal source compound for preparing the catalyst have to be appropriately adjusted so that the molar ratio of the respective elements in the obtained catalyst falls within a desired range. The molar ratio of the respective elements is determined by the used amounts (blended amounts) of the raw compounds. In the case of preparing the catalyst containing boron phosphate, the used amounts of the phosphate compound and the boron compound have to be appropriately adjusted so that the molar ratio P/B in the obtained catalyst falls within the prescribed range. In the case of preparing the catalyst containing the rare-earth metal phosphate, the used amounts of the phosphate compound and the rare-earth metal compound have to be appropriately adjusted so that the molar ratio P/R in the obtained catalyst falls within the prescribed range. In the case of preparing the catalyst containing boron phosphate and the metal element, the used amounts of the phosphate compound, the boron compound and the metal source compound have to be appropriately adjusted so that the molar ratio M/(P+B) in the obtained catalyst falls within the prescribed range. In the case of preparing the catalyst containing the rare-earth metal phosphate and the metal element, the used amounts of the phosphate compound, the rare-earth metal compound and the metal source compound have to be appropriately adjusted so that the molar ratio M/(P+R) in the obtained catalyst falls within the prescribed range.

As the phosphate compound used for the raw compound of the catalyst, phosphoric acid such as $H_3PO_2$, $H_3PO_3$, $H_3PO_4$, $H_4P_2O_7$, $H_5P_3O_{10}$ or $H_6P_4O_{10}$; a phosphate ester such as trim ethyl phosphate and triethyl phosphate; an ammonium phosphate such as ammonium dihydrogenphosphate, diammonium hydrogenphosphate or triammonium phosphate; or a phosphorous oxide such as $P_4O_6$, $P_4O_8$, $P_4O_9$ or $P_4O_{10}$ may be used. These phosphate compounds may be used either only one kind or in a combination of at least two kinds of them. The preferable forms of these phosphate compounds may be appropriately adopted according to the preparation method.

As the boron compound used for the raw compound of the catalyst, $H_3BO_3$, $HBO_3$, $H_4B_2O_4$, $H_3BO_2$, $H_3BO$, $(NH_4)_2O.5B_2O_3.8H_2O$ or the like may be used. These boron compounds may be used either only one kind or in a combination of at least two kinds of them. The preferable forms of these boron compounds may be appropriately adopted according to the preparation method.

As the rare-earth metal compound used for the raw material of the catalyst, a rare-earth metal oxide; a rare-earth metal hydroxide including a dehydration condensate of a rare-earth metal hydroxide; an inorganic rare-earth metal salt such as nitrate, carbonate, chloride, bromide or iodide; or a rare-earth metal salt of an organic acid such as formate, acetate, oxalate or citrate may be used. These rare-earth metal compounds may be used either only one kind or in a combination of at least two kinds of them. The preferable forms of these rare-earth metal compounds may be appropriately adopted according to the preparation method.

As the metal source compound used for the raw compound of the catalyst, a metal oxide; a metal hydroxide, an inorganic metal salt such as nitrate, carbonate, chloride, bromide or iodide; or a metal salt of an organic acid such as formate, acetate, oxalate or citrate may be used. These metal source compounds may be used either only one kind or in a combination of at least two kinds of them. The preferable forms of these metal source compounds may be appropriately adopted according to the preparation method.

For example, in the case of preparing the catalyst comprising boron phosphate or the catalyst comprising boron phosphate and the metal element by using the condensation method or the kneading method, it is preferred that a phosphoric acid such as $H_3PO_2$, $H_3PO_3$, $H_3PO_4$, $H_4P_2O_7$, $H_5P_3O_{10}$ or $H_6P_4O_{10}$, or an ammonium phosphate such as ammonium dihydrogenphosphate, diammonium hydrogenphosphate or triammonium phosphate is used as the phosphate compound, $H_3BO_3$, $HBO_3$, $H_4B_2O_4$, $H_3BO_2$, $H_3BO$, $(NH_4)_2O.5B_2O_3.8H_2O$ or the like is use as the boron compound, and a metal hydroxide, a metal nitrate or metal carbonate is used as the metal source compound. When such compounds are used, the raw compounds are easily obtained and the catalyst containing fewer impurities is easily prepared.

For example, in the case of preparing the catalyst comprising the rare-earth metal phosphate or the catalyst comprising the rare-earth metal phosphate and the metal element by using the kneading method, it is preferred that an ammonium phosphate such as ammonium dihydrogenphosphate, diammonium hydrogenphosphate or triammonium phosphate is used as the phosphate compound, a rare-earth metal oxide, a rare-earth metal hydroxide, a rare-earth metal nitrate or the like is use as the rare-earth metal compound, and a metal hydroxide, a metal nitrate or a metal carbonate is used as the metal source compound. When such compounds are used, the raw compounds are easily obtained, kneading operation is easily conducted, and the catalyst containing fewer impurities is easily prepared.

For example, in the case of preparing the catalyst comprising the rare-earth metal phosphate or the catalyst comprising the rare-earth metal phosphate and the metal element by using the sol-gel method, it is preferred that phosphoric acid such as $H_3PO_2$, $H_3PO_3$, $H_3PO_4$, $H_4P_2O_7$, $H_5P_3O_{10}$ or $H_6P_4O_{10}$, or an ammonium phosphate such as ammonium dihydrogenphosphate, diammonium hydrogenphosphate or triammonium phosphate is used as the phosphate compound, and a rare-earth metal hydroxide including a dehydration condensate of a rare-earth metal hydroxide is used as the rare-earth metal compound. When such compounds are used, sol- or gel-like material containing phosphate and a rare-earth metal is easily prepared by adding the phosphate compound into a solution containing a rare-earth metal hydroxide.

In the sol-gel method, addition rate and temperature in adding the phosphate compound into the solution containing a rare-earth metal hydroxide is not particularly limited. The temperature in adding may be usually in the range of 0°C to 120°C. The resultant obtained by adding the phosphate compound to the solution containing a rare-earth metal hydroxide is preferably left as it is. During the resultant being left, the formed amount of the sol- or gel-like material containing phosphate and the rare-earth metal is increased.

The rare-earth metal hydroxide, that is preferably used in the sol-gel method, may be prepared as follows. A water-soluble rare-earth metal salt such as an inorganic rare-earth metal salt or a rare-earth metal salt of an organic acid is added into a solvent containing water, thereby preparing the rare-earth metal hydroxide. On this occasion, it is preferred that an alkaline compound such as ammonia or an amine is added into the solvent containing water in addition to the rare-earth metal salt to make the pH of the solvent in the range of 2 to 13 (preferably in the range of 4 to 11, and more preferably in the range of 7 to 9). As the solvent containing water, a solvent containing only water is preferably used, since it is easy-to-use and economical. An amount of the rare-earth metal salt which is added into the solvent is preferably 1 mass% or more and 30 mass% or less, more preferably 2 mass% or more and 20 mass% or less, and even more preferably 3 mass% or more and 15 mass% or less, relative to 100 mass% of total amounts of the solvent and the rare-earth metal salt.

Examples of the alkaline compound used in the preparation of the rare-earth metal hydroxide include ammonia; aliphatic amines such as methyl amine, ethyl amine, n-propyl amine, isopropyl amine, sec-butyl amine, dimethyl amine, diethyl amine, trimethyl amine and triethyl amine; alicyclic amines such as cyclohexyl amine; alkanol amines such as monoethanol amine, diethanol amine and triethanol amine; pyridine; ammonium carbonate; and urea. These alkaline compounds may be used either only one kind or in a combination of at least two kinds of them. Among them, ammonia is particularly preferred.

In adding the alkaline compound into the solution which has been prepared by adding the rare-earth metal salt into the solvent containing water, the alkaline compound may be added little by little or may be added at once; however, the alkaline compound is preferably added little by little in order to make uniform the particle size of the rare-earth metal hydroxide. The temperature of the solution when adding the alkaline compound is not particularly limited; however, taking into consideration the difficulty in pH adjustment due to the evaporation of a volatile alkaline compound when the selected alkaline compound is volatile and the easily formation of the rare-earth metal hydroxide, it is generally in the range of 0°C to 120°C, preferably in the range of 20°C to 50°C. After the completion of adding the alkaline compound, the resultant is preferred to be left as it is without the phosphate compound being immediately added thereto. During the resultant being left, particles of the rare-earth metal hydroxide are grown and the sizes of the particles become uniform.

In the case of preparing the catalyst comprising the metal element by the sol-gel method, a metal hydroxide, a metal nitrate, a metal carbonate, a metal acetate, a metal oxalate or the like is preferably used, and a metal hydroxide, a metal nitrate, a metal carbonate or the like is more preferably used, as the metal source compound. In this case, timing of adding the metal source compound is not particularly restricted. The metal source compound may be added to the solution, which has been prepared by adding the rare-earth metal salt into the solvent containing water, before or after the addition of the alkaline compound or at the same time as the addition of the alkaline compound. Alternatively, the metal source solution may be added to the solution containing the rare-earth metal hydroxide before or after the addition of the phosphate compound or at the same time as the addition of the phosphate compound. Still alternatively, the metal source compound may be added to the calcinated product prepared by calcinating the sol- or gel-like material containing phosphate and the rare-earth metal, where the sol- or gel-like material may be subjected to a dehydration treatment or a drying treatment in advance of the calcination. In this case, the calcinated product to which the metal source compound has been added is preferably re-calcinated.

The solid matter (catalyst precursor) obtained by kneading the raw compounds, the solid matter (catalyst precursor) obtained by adding raw compounds into the solvent, or the material (catalyst precursor) formed by adding the metal source compound to the calcinated product which has been prepared by calcinating the solid matter formed by adding the raw materials without containing the metal source compound into the solvent, is calcinated, thereby obtaining the catalyst of the present invention. In the calcination of the catalyst precursor, crystallizing of the boron phosphate or the rare-earth metal phosphate to be contained in the catalyst tends to be promoted as the calcination temperature is higher and the calcination time is longer. Therefore, taking those trends into consideration, the calcination conditions may be appropriately determined to obtain the catalyst comprising the boron phosphate or the rare-earth metal phosphate having a crystalline structure (in which catalyst may further comprise the metal element). The calcination is preferably conducted, for example, under air atmosphere at the temperature of 500°C to 1500°C for from 3 hours to 15 hours, more preferably at the temperature of 600°C to 1400°C for from 3 hours to 10 hours, and even more preferably at the temperature of 700°C to 1200°C for from 3 hours to 5 hours.

The catalyst precursor may be subjected to a pre-heat treatment in advance of the calcination. For example, in the case where the catalyst precursor contains an ammonium component or a nitrate component, if the catalyst precursor is calcinated without being subjected to the pre-heat treatment, a gas derived from the ammonium component or the nitrate component may possibly generate, resulting in scattering the catalyst precursor or the catalyst, or inducing the explosion. Therefore, to decrease the gas generated at the calcination, the catalyst precursor may be subjected to the pre-heat treatment in advance of the calcination. In the pre-heat treatment, for example, the catalyst precursor may be placed in air atmosphere or inert gas atmosphere at the temperature of 150°C to 450°C. Further, the catalyst precursor may be subjected to a drying treatment for removing water from the catalyst precursor in advance of the calcination or pre-heat of the catalyst precursor.

In the case where boron phosphate or the rare-earth metal phosphate is supported on a carrier, an impregnation method where a carrier is impregnated with a solution containing the raw compounds of boron phosphate or the rare-earth metal phosphate and heated; a deposition precipitation method where boron phosphate or the rare-earth metal phosphate is made deposited in a solution containing a carrier, or a carrier is added into a solution in which boron phosphate or the rare-earth metal phosphate is precipitated; a kneading method where boron phosphate or the rare-earth metal phosphate is mixed with a carrier, or the catalyst precursor is mixed with a carrier; or the like may be employed. In the case where the metal element is also supported on the carrier, the above preparing method may be conducted while adding the metal source compound in addition to boron phosphate or the rare-earth metal phosphate.

According to the above method, the catalyst for glycerin dehydration can be prepared. Thus prepared glycerin dehydration catalyst is useful as a catalyst used for dehydrating glycerin. Accordingly, the catalyst for glycerin dehydration of the present invention can be used for a process for producing acrolein by dehydrating glycerin.

[Process for producing acrolein]

A process for producing acrolein of the present invention is explained. A process for producing acrolein of the present invention comprises the step of dehydrating glycerin in the presence of the catalyst of the present invention to produce acrolein.

In the producing process of the present invention, acrolein is produced, for example, by gas-phase dehydration reaction in which a reaction gas containing glycerin gas is brought into contact with the catalyst in any reactor selected from a fixed-bed reactor, a fluidized-bed reactor, a moving-bed reactor, and the like. However, the producing process of the present invention is not limited to the gas-phase dehydration reaction in which the reaction gas containing glycerin gas is brought into contact with catalyst, and is also capable of applying to liquid-phase dehydration reaction in which glycerin solution is brought into contact with the catalyst. In the latter case, the liquid-phase dehydration reaction can be conducted by using various known methods such as a method with a combination of a fixed-bed and a distillation column, a method with a combination of a stirred vessel and a distillation column, a method of using a single-stage stirred vessel, a method of using a multiple-stage stirred vessel, a method of using a multiple-stage distillation column, and method with combination of them. These method may be conducted either batch-wise or continuously, and is usually conducted continuously.

Glycerin, a synthetic raw material of acrolein, is not particularly limited, and may be either purified glycerin or crude glycerin. The glycerin may be derived from natural resources, that is, for example, glycerin may be obtained by ester exchange reaction of a vegetable oil such as palm oil, palm kernel oil, coconut oil, soybean oil, rape seed oil, olive oil, or sesame oil; or glycerin may be obtained by ester exchange reaction of an animal fat or oil such as fish oil, beef tallow, lard, or whale oil. There may also be used glycerin chemically synthesized from ethylene, propylene, or the like.

The following describes, as an example, a process for producing acrolein by gas-phase dehydration reaction, which is excellent in the industrial productivity.

The reaction gas to be introduced into a catalyst layer where the catalyst for glycerin dehydration is filled may consist of only glycerin or may further contain a gas which is inactive to the dehydration reaction of glycerin to adjust glycerin concentration in the reaction gas. Examples of the inactive gas include, for example, steam, nitrogen gas, carbon dioxide gas, air, and the like. The glycerin concentration in the reaction gas is generally in the range of 0.1 mol% to 100 mol%, preferably 1 mol% of more, and more preferably 5 mol% or more for economically and efficiently producing acrolein.

A flow rate of the reaction gas, a gas space velocity per unit volume of the catalyst (GHSV), is generally in the range of 50 h$^{-1}$ to 20000 h$^{-1}$, preferably 10000 h$^{-1}$ or lower, and more preferably 4000 h$^{-1}$ or lower for economically and efficiently producing acrolein.

In the gas-phase dehydration reaction of glycerin, if the reaction temperature is too low or too high, yield of acrolein decreases; therefore, the reaction temperature is generally in the range of 200°C to 500°C, preferably in the range of 250°C to 450°C, and more preferably in the range of 300°C to 400°C. The "reaction temperature" in the gas-phase dehydration reaction as used herein means a preset temperature of a heat medium or the like which control the temperature of a reactor.

A pressure of the reaction gas is not particularly limited as long as it is in the range where glycerin does not condense, and is generally in the range of 0.001 MPa to 1 MPa, preferably in the range of 0.01 MPa to 0.5 MPa, more preferably 0.3 MPa or lower, and particularly preferably 0.2 MPa or lower.

When the dehydration reaction of acrolein is continuously conducted, carbonaceous matters may be deposited on the surface of the catalyst, resulting in decreasing the activity of the catalyst. Specifically, selectivity of acrolein is lowered and selectivity of propionaldehyde is enhanced. In such a case, when a regeneration treatment in which the catalyst is brought into contact with a regeneration gas at high temperature is conducted, the carbonaceous matters deposited on the surface of the catalyst can be removed, thereby regenerating the activity of the catalyst. Examples of the regeneration gas include, for example, oxidative gases such as oxygen and air which contains oxygen. The regeneration gas may further contain an inert gas which is inactive against the regeneration treatment, such as nitrogen, carbon dioxide or steam, if needed. In the case where there is a risk of abrupt heat generation due to contact of the catalyst with oxygen, it is recommended that the inert gas is contained in the regeneration gas for suppressing the abrupt heat generation. Temperature of the regeneration treatment is not particularly limited as long as the carbonaceous matters can be removed without occurring heat deterioration of the catalyst, and is preferably equal to or lower than calcination temperature in preparing the catalyst.

An acrolein-containing gas obtained by the dehydration reaction of glycerin may be supplied as a reaction gas for producing acrylic acid without being purified; however, since the acrolein-containing gas contains by-products, it is preferred to be purified. Examples of the by-product include, for example, phenol, 1-hydroxyacetone, and allyl alcohol in addition to propionaldehyde. In the case of using glycerin derived from biodiesel as a raw material, examples of the by-product include, for example, phenol, 1-hydroxyacetone, methoxyacetone and 3-methoxypropanal. Using acrolein accompanied with such compounds as by-products for producing acrylic acid causes the production of by-products such as formic acid, acetic acid, propionic acid, pyruvic acid or 3-methoxypropionic acid in acrylic acid.

In the case where the acrolein-containing gas is purified, the acrolein-containing gas may be directly subjected to purification step such as distillation, or may be collected as crude acrolein once and then subjected to purification step. Examples of methods for collecting the acrolein-containing gas include a method of cooling the acrolein-containing gas to condense, and a method of having the acrolein-containing gas absorbed by an acrolein-soluble solvent such as water. For example, a method using a purification apparatus provided with a collection column and a diffusion column, which is disclosed in Japanese Unexamined Patent Application Publication No. 2008-115103 is employed. In the case where acrolein is obtained as a gaseous substance in the purification step, the purified acrolein gas may be supplied as a reaction gas of acrylic acid production, or may be once collected as purified acrolein and then the purified acrolein is utilized for acrylic acid production.

In the purification of the crude acrolein, phenol and/or 1-hydroxyacton is mainly removed. When these by-products are removed, yield of acrylic acid is enhanced in producing acrylic acid from acrolein. Specifically, product amount of acetic acid can be reduced by removing 1-hydroxyaceton.

In consideration of enhancing the yield of acrylic acid, it is considered to be preferable that a larger amount of phenol and/or 1-hydroxyacton is removed. Thus, a mass ratio Ph/A of phenol (Ph) to acrolein (A) after the purification is preferably 1.0 or less, more preferably 0.7 or less, and even more preferably 0.4 or less. In addition, a mass ratio H/A of 1-hydroxyacetone (H) to acrolein (A) after the purification is preferably 0.5 or less, more preferably 0.3 or less, and even more preferably 0.1 or less. Meanwhile, when a further larger amount of phenol and/or 1-hydroxyacton is removed, loss of acrolein may be increased or the purification of acrolein may be complicated. Taking these facts into consideration, the mass ratios Ph/A and H/A are preferably $1 \times 10^{-9}$ or more, more preferably $1 \times 10^{-7}$ or more, and even more preferably $1 \times 10^{-5}$ or more.

Boiling points of acrolein, phenol and 1-hydroxyacetone are about 53°C, about 182°C and about 146°C, respectively. By utilizing the differences between theses boiling points, phenol and/or 1-hydroxyacetone can be removed from the crude acrolein. Examples of method for that include, for example, a method of fractional-distilling acrolein having a lower boiling point than removal objectives by treating the liquid crude acrolein with a distillation column, a method of condensing removal objectives having higher boiling points than acrolein by treating the gaseous crude acrolein with a condensation column, and a method of vaporizing acrolein having a lower boiling point than removal objectives by blowing a gas into the crude acrolein introduced into a diffusion column.

In addition, melting points of acrolein, phenol and 1-hydroxyacetone are about -87°C, about 43°C and about -17°C, respectively. By utilizing the differences between theses melting points, phenol and/or 1-hydroxyacetone can be removed from the crude acrolein. Examples of method for that include, for example, a method of removing crystals of phenol and/or 1-hydroxyacetone by cooling the crude acrolein.

Propionaldehyde has a boiling point of about 48°C and a melting point of about -81 °C, and thus, it is possible to remove propionaldehyde from the crude acrolein by utilizing the difference of the boiling or melting points between propionaldehyde and acrolein. However, since the both differences of the boiling point and the melting point between propionaldehyde and acrolein are small, loss of acrolein may possibly increase. Therefore, the catalyst of the present invention particularly useful, since the production of propionaldehyde can be reduced in the dehydration reaction of glycerin.

In the case where the crude acrolein is used for a synthetic raw material of other compound, the crude acrolein does not need to be subjected to the purification. For example, in the case where acrylic acid is produced from the crude acrolein, impurities in the acrylic acid may be removed by purifying the acrylic acid in the subsequent step while not purifying the crude acrolein. In view of simplifying the process and lowering the production cost, it is preferred that the crude acrolein is not purified to be used.

According to the above, acrolein can be produced. The produced acrolein is useful as a synthetic raw material of acrolein derivatives such as acrylic acid, 1,3-propanediol, allyl alcohol and methionine; hydrophilic resins such as polyacrylic acid and sodium polyacrylate; and the like, as is already well-known. Therefore, it is possible, of course, that the process for producing acrolein of the present invention is incorporated into processes for producing acrolein derivatives and hydrophilic resins.

[Process for producing acrylic acid]

A process for producing acrylic acid of the present invention is explained. A process for producing acrylic acid of the present invention comprises the step of oxidizing acrolein produced by the process for producing acrolein of the above to produce acrylic acid. That is, the process for producing acrylic acid of the present invention comprises the steps of: dehydrating glycerin in the presence of the catalyst of the present invention to produce acrolein; and oxidizing the acrolein to produce acrylic acid. Thus, acrolein obtained by the process for producing acrolein of the present invention can be utilized as a raw material of acrylic acid.

For producing acrylic acid, it is preferred that a gas containing acrolein, that may be hereinafter referred to as an "acrolein-containing gas", and a catalyst for oxidizing acrolein, that may be hereinafter referred to as a "catalyst for acrolein oxidation", are made coexisted in any oxidation reactor selected from a fixed-bed reactor, a moving-bed reactor, a fluidized-bed reactor, and the like, and acrolein is oxidized in a gas phase at temperature of 200°C to 400°C. On this occasion, propionic acid is produced from propionaldehyde that accompanies oxidation of acrolein; however, since acrolein obtained by using the catalyst of the present invention is accompanied by lesser amount of propionaldehyde, production amount of propionic acid is small.

The catalyst for oxidizing acrolein is not particularly limited as long as it is a conventionally-known catalyst for acrolein oxidation that is used for producing acrylic acid by gas-phase catalytic oxidation of acrolein with molecular oxygen or a gas containing molecular oxygen. Examples of the catalyst may include, for example, mixtures of metal oxides such as iron oxide, molybdenum oxide, titanium oxide, vanadium oxide, tungsten oxide, antimony oxide, tin oxide, and copper oxide; and complex oxides of these metal oxides. Among these catalysts, a molybdenum-vanadium catalyst containing molybdenum and vanadium as main components is particularly suitable. Further, the catalyst may be a supported catalyst in which the above mixture of metal oxides or the complex oxide is supported on a carrier (e.g., inorganic oxide such as silica, alumina, and zirconia, complex oxide of them, or an inorganic compound such as silicon carbide).

With respect to the amount of oxygen to be added to the acrolein-containing gas used in the production of acrylic acid, when the amount of oxygen is excess, there may be a risk of explosion due to the combustion of acrolein; and therefore, it follows that the upper limit thereof should be set appropriately.

By the gas-phase oxidation of acrolein, a gaseous substance containing crude acrylic acid is obtained. The gaseous substance is liquefied by cold condensation, collection using a solvent, or the like, and from the obtained liquefied substance, water or the collection solvent is removed by a conventionally-known method (e.g., distillation), if necessary, and then crystallization operation is conducted, thereby enabling the production of high-purity acrylic acid.

The crude acrylic acid obtained by oxidation of acrolein contains propionic acid as a by-product. The content of propionic acid in the crude acrylic acid is relatively small, since the catalyst of the present invention is used in the step of producing acrolein, a raw material of acrylic acid. Nevertheless, in the case of producing a water-absorbent resin from the acrylic acid, it is preferred that propionic acid is removed from the crude acrylic acid because propionic acid causes odor. Therefore, the crude acrylic acid is purified to remove propionic acid.

Both boiling points of acrylic acid and propionic acid are about 141 °C. Therefore, it is difficult to remove propionic acid from the crude acrylic acid by utilizing the differences between these boiling points. Meanwhile, melting points of acrylic acid and propionic acid are about 12°C and about -21°C, respectively. Therefore, it is easy to remove propionic acid from the crude acrylic acid by utilizing the differences between these melting points. Thus, for removing propionic acid from the crude acrylic acid, it is proper that the crude acrylic acid is subjected to the crystallization operation. Specifically, the crude acrylic acid is cooled to recover acrylic acid which crystallizes in advance of propionic acid. In this case, cooling temperature of the crude acrylic acid is preferably in the range of -18°C to 10°C, more preferably 4°C or lower, and even more preferably 0°C or lower. In the case where the crude acrylic acid contains impurities other than propionic acid, such as acetic acid, acrolein or water, it is preferred that these impurities are removed by a conventionally-known method such as distillation, followed by removing propionic acid by the crystallization operation.

The crystallization operation is not particularly restricted as long as it can separate propionic acid from crude acrylic acid, and can be conducted by a conventionally-known method disclosed in, for example, Japanese Unexamined Patent Application Publication Nos. 9-227445 and 2002-519402.

The crystallization operation is the step of crystallizing acrylic acid by supplying crude acrylic acid to a crystallization apparatus to obtain purified acrylic acid. As a method of crystallization, a conventionally-known crystallization method can be employed, and the method is not particularly restricted; however, the crystallization may be performed in one or more stage(s) by using a continuous or batch type crystallization apparatus. If necessary, the obtained crystallized acrylic acid may be further subjected to purification such as washing or sweating to obtain purified acrylic acid with further improved purity.

Examples of the continuous crystallization apparatus include a crystallization apparatus in which a crystallization part, a solid-liquid separation part and a crystal purification part are united (e.g., BMC (Backmixing Column Crystallizer) manufactured by Nippon Steel Chemical Co., Ltd.; continuous melting and purifying system manufactured by Tsukishima Kikai Co., Ltd.), and a crystallization apparatus in combination with a crystallization part (e.g., CDC (Cooling Disc Crystallizer) manufactured by GMF GOUDA), a solid-liquid separation part (e.g., a centrifugal separator or a belt filter), and a crystal purification part (e.g., KCP (Kureha Crystal Purifier) manufactured by Kureha Engineering Co., Ltd.).

Examples of the batch type crystallization apparatus include a layer-crystallizing apparatus (a dynamic crystallization apparatus) manufactured by Sulzer Chemtech Ltd. and a static crystallization apparatus manufactured by BEFS PROKEM.

Dynamic crystallization is a method for conducting crystallization by using a dynamic crystallization apparatus which is equipped with, for example, a tubular crystallizer provided with a temperature control mechanism for conducting crystallizing, sweating and melting, a tank for recovering a mother liquid after sweating, and a circulation pump for supplying crude acrylic acid to the crystallizer, and in which the crude acrylic acid is transferred by the circulation pump from a storage tank installed under the crystallizer to an upper part of the tubular crystallizer. Meanwhile, static crystallization is a method for conducting crystallization by using a static crystallization apparatus which is equipped with, for example, a tubular crystallizer provided with a temperature control mechanism for conducting crystallizing, sweating and melting, wherein the crystallizer has a discharge valve at a lower part thereof, and a tank for recovering a mother liquid after sweating.

Specifically, crude acrylic acid of a liquid phase is fed to a crystallizer, whereby acrylic acid in the liquid phase is solidified to be deposited on a cooling face (a tubular wall face). When the mass of the solid deposited on the cooling face reaches preferably 10 mass% to 90 mass%, and more preferably 20 mass% to 80 mass%, of the crude acrylic acid fed to the crystallizer, the liquid is immediately discharged out of the crystallizer to separate the solid and the liquid. The liquid may be discharged by either of a method of pumping up the liquid (dynamic crystallization) or a method of flowing the liquid out of the crystallizer (static crystallization). Meanwhile, the solid is taken out of the crystallizer, and then, may be purified by washing or sweating for further improving the purity.

In the case where the dynamic crystallization or the static crystallization is performed in multistage, the crystallization can be conducted advantageously by employing the counter-flow principle. In this case, the crystallized acrylic acid is separated from the remaining mother liquid in each stage and supplied to the stage in which acrylic acid with higher purity is produced. Meanwhile, the remaining mother liquid is supplied to the stage in which acrylic acid with lower purity is produced.

In the dynamic crystallization, when the purity of acrylic acid is low, crystallization becomes difficult; whereas, in the static crystallization, crystallization is easily performed even when the purity of acrylic acid is low, since contact time of the remaining mother liquid with the cooling face is prolonged and effect of the temperature tends to be transferred easily, compared with the dynamic crystallization. Therefore, for improving recovery efficiency of acrylic acid, the final remaining mother liquid of the dynamic crystallization may be subjected to the static crystallization to be further crystallized.

The number of stages for the crystallization to be needed depends on the demand of the degree of purity, and the number of stages needed for obtaining acrylic acid with high purity is generally 1 to 6 stages, preferably 2 to 5 stages, more preferably 2 to 4 stages for purification stage (in the dynamic crystallization) and generally 0 to 5 stages, preferably 0 to 3 stages for stripping stage (in the dynamic crystallization and/or the static crystallization). In general, stages in which acrylic acid with higher purity than that of supplied crude acrylic acid is obtained are all purification stages, and stages other than these stages are all stripping stages. The stripping stage is conducted for recovering acrylic acid contained in the remaining mother liquids generated from the purification stages. The striping stage is not necessarily provided, and, in the case where low boiling point components are separated from the remaining mother liquid of the crystallization apparatus by using a distillation column, for example, the stripping stage may be omitted.

Even in the case of employing either the dynamic crystallization or the static crystallization, the crystallized acrylic acid obtained in the crystallization operation may be used as-is as a product, or may be further purified by washing or sweating to obtain a product, if necessary. On the other hand, the remaining mother liquid discharged from the crystallization operation may be taken out of the system.

According to the above, acrylic acid can be produced. The produced acrylic acid is useful as a synthetic raw material of acrylic acid derivatives such as acrylic acid ester; hydrophilic resins such as polyacrylic acid and sodium polyacrylate; and the like, as is already well-known. Therefore, it is possible, of course, that the process for producing acrylic acid of the present invention is incorporated into processes for producing acrylic acid derivatives and hydrophilic resins.

[Process for producing hydrophilic resin]

A process for producing a hydrophilic resin of the present invention comprises the step of polymerizing a monomeric component including acrylic acid produced by the process for producing acrylic acid of the above. That is, the process for producing a hydrophilic resin of the present invention comprises the steps of: dehydrating glycerin in the presence of the catalyst of the present invention to produce acrolein; oxidizing the acrolein to produce acrylic acid; and polymerizing a monomeric component including the acrylic acid. Thus, acrylic acid obtained by the process for producing acrylic acid of the present invention can be utilized as a raw material of a hydrophilic resin such as a water-absorbent resin and a water-soluble resin.

Acrylic acid obtained by the process for producing acrylic acid from glycerin contains a large amount of impurities of organic acids such as formic acid, acetic acid or propionic acid as byproducts, compared with acrylic acid obtained by a process for producing acrylic acid from propylene, and these impurities possibly cause odor or coloration of the hydrophilic resin. Thus, it is important to purify the obtained acrylic acid. Among the impurities contained in the acrylic acid, propionic acid has similar boiling point to acrylic acid, and therefore, when propionic acid is contained in a large amount, purification of acrylic acid by distillation becomes difficult. Accordingly, in the process for producing a hydrophilic resin of the present invention, acrylic acid from which propionic acid has been removed by purification of crystallization is preferably used.

When acrylic acid obtained by the process for producing acrylic acid of the present is used as a raw material for producing hydrophilic resins such as a water-absorbent resin and a water-soluble resin, polymerization reaction is easily controlled, quality of the obtained hydrophilic resin becomes stabilized, and various performances such as absorption ability and dispersion performance of an inorganic material are improved.

For producing the water-absorbent resin, for example, acrylic acid and/or its salt obtained by the process for producing acrylic acid of the present invention is used as a main component (preferably 70 mol% or more, and more preferably 90 mol% or more) of monomeric components, and further about 0.001 mol% to 5 mol% (value relative to acrylic acid) of a crosslinking agent and about 0.001 mol% to 2 mol% (value relative to the monomeric components) of a radical polymerization initiator are used to conduct crosslinking polymerization, and then the product is dried and pulverized to obtain the water-absorbent resin.

The water-absorbent resin means water-swellable and water-insoluble polyacrylic acid having a crosslinked structure, which forms water-insoluble hydrogel containing preferably 25 mass% or less, more preferably 10 mass% or less of a water-soluble component (a water-soluble fraction) by absorbing deionized water or normal saline solution in an amount of 3 times or more, preferably 10 times to 1000 times as much as the weight of the polymer itself. Specific examples and measurement methods of physical properties of the water-absorbent resin like this is described in U.S. Patent Publications Nos. 6,107,358, 6,174,978, 6,241,928 and the like.

Preferable producing method in view of improving productivity are disclosed in, for example, U.S. Patent Publications Nos. 6,867,269, 6,906,159 and 7,091,253, International Publications Nos. WO 01/038402 and WO 2006/034806, and the like.

A sequence of the steps for producing the water-absorbent resin from acrylic acid as a starting raw material by neutralization, polymerization, drying and the like are, for example, as follows.

A part or all of acrylic acid obtained by the producing process of the present invention is supplied to the producing process of the water-absorbent resin through a line. In the producing process of the water-absorbent resin, acrylic acid is introduced into a neutralization step, a polymerization step and a drying step to be conducted desired treatments, thereby producing the water-absorbent resin. For improving various physical properties, any treatments may be conducted, and, for example, a crosslinking step may be conducted during the polymerization or after the polymerization.

The neutralization step is optional, and, for example, a method of mixing a powder or aqueous solution of a predetermined amount of a basic substance with acrylic acid or polyacrylic acid (salt) is exemplified; however, a conventionally-known method may be employed without any limitation. The neutralization step may be conducted either before the polymerization or after the polymerization or both before and after the polymerization. As the basic substance to be used for neutralizing acrylic acid or polyacrylic acid (salt), conventionally-known basic substances such as, for example, (hydrogen) carbonates, alkali metal hydroxides, ammonia, or organic amines may be used appropriately. Neutralization ratio of the polyacrylic acid is not particularly limited and may be adjusted to be an optional neutralization ratio (e.g., an optional value in the range of 30 mol% to 100 mol%).

A polymerization method in the polymerization step is not particularly limited, and a conventionally-known polymerization method such as polymerization using a radical polymerization initiator, radiation polymerization, polymerization by radiating electron beam or active energy beam, or ultraviolet polymerization using a photosensitizer may be employed. Various conditions such as kinds of the polymerization initiator and polymerization conditions may be adopted appropriately. Of course, conventionally-known additives such as crosslinking agents, other monomers, water-soluble chain transfer agents and hydrophilic polymers can be added, if necessary.

Examples of the polymerization method using a radical polymerization initiator include, for example, an aqueous solution polymerization method and a reverse-phase suspension polymerization method. The aqueous solution polymerization method is a method in which acrylic acid in an aqueous solution of acrylic acid is polymerized without using any dispersion solvent and the like, and is disclosed in U.S. Patent Publications Nos. 4,625,001, 4,873,299, 4,286,082, 4,973,632, 4,985,518, 5,124,416, 5,250,640, 5,264,495, 5,145,906, and 5,380,808, and European Patent Publications Nos. 0 811 636, 0 955 086, and 0 922 717. The reverse-phase suspension polymerization method is a polymerization method in which acrylic acid in an aqueous solution of acrylic acid is polymerized in the state where the aqueous solution of acrylic acid is suspended in a hydrophobic organic solvent, and is disclosed in U.S. Patent Publications Nos. 4,093,776, 4,367,323, 4,446,261, 4,683,274, and 5,244,735.

Acrylate polymer obtained by the polymerization, that is, water-absorbent resin, is subjected to a drying step. A drying method is not particularly limited, and a conventionally-known drying means such as a hot-air drier, a fluidized-bed drier, and a Nauta drier may be used for appropriately drying at a desired drying temperature, preferably at a temperature in the range of 70°C to 230°C.

The water-absorbent resin obtained in the drying step may be used as-is, or may be used after granulation and pulverization into a desired shape and surface-crosslinking. Further, the water-absorbent resin may be subjected to post-treatments corresponding to the usage such as adding conventionally-known additives including a reducing agent, a fragrance and a binder.

EXAMPLES

The present invention is hereinafter described more specifically by reference to Examples; however, the present invention is not limited to these Examples, and can be put into practice after appropriate modifications or variations within a range meeting the gist of the present invention, all of which are included in the technical scope of the present invention.

(1) Catalyst comprising boron phosphate

(1-1) Preparation of catalyst (Preparation of catalyst by a condensation method)

Example A1

Into an aqueous solution consisting of 169.67 g of boric acid and 1567.5 g of distilled water, a solution consisting of 332.17 g of 85% phosphoric acid aqueous solution and 232.52 g of distilled water was added, to give a mixed solution. The mixed solution was heated to reflux at 90°C for 2 hours while stirring, thereby obtaining a clear, colorless mixed solution. The thus obtained mixed solution was dewatered to condense on a hot-water bath of 60°C under reduced pressure condition of 0.005 MPa by using an evaporator. Thus obtained condensed matter was dried at 120°C under airflow for 24 hours to obtain a solid matter. The solid matter was calcinated at 1000°C under airflow for 5 hours. Thus obtained calcinated product was sieved by using sieves with opening apertures of 0.7 mm or 2.0 mm, thereby obtaining a classified calcinated product with the size of 0.7 mm to 2.0 mm as a catalyst. The obtained catalyst was a catalyst for glycerin dehydration comprising boron phosphate.

Example A2

A catalyst was prepared in the same manner as in Example A1, except using the mixed solution obtained by adding a solution consisting of 337.05 g of 85% phosphoric acid aqueous solution and 235.93 g of distilled water into a solution consisting of 164.34 g of boric acid and 1564.1 g of distilled water in Example A1.

Example A3

A catalyst was prepared in the same manner as in Example A1, except using the mixed solution obtained by adding a solution consisting of 345.94 g of 85% phosphoric acid aqueous solution and 242.16 g of distilled water into a solution consisting of 154.61 g of boric acid and 1557.8 g of distilled water in Example A1.

Example A4

A catalyst was prepared in the same manner as in Example A1, except using the mixed solution obtained by adding a solution consisting of 367.25 g of 85% phosphoric acid aqueous solution and 257.07 g of distilled water into a solution consisting of 131.31 g of boric acid and 1542.9 g of distilled water in Example A1.

Comparative Example A1

A catalyst was prepared in the same manner as in Example A1, except using the mixed solution obtained by adding a solution consisting of 306.32 g of 85% phosphoric acid aqueous solution and 214.42 g of distilled water into a solution consisting of 197.94 g of boric acid and 1585.6 g of distilled water in Example A1.

Comparative Example A2

A catalyst was prepared in the same manner as in Example A1, except using the mixed solution obtained by adding a solution consisting of 326.96 g of 85% phosphoric acid aqueous solution and 228.87 g of distilled water into a solution consisting of 175.36 g of boric acid and 1571.1 g of distilled water in Example A1.

(1-2) Examples of acrolein production (initial performance)

Glycerin was dehydrated to produce acrolein by the following atmospheric current gas-phase fixed-bed reaction, using each of the catalysts prepared in the above Examples A1 to A4 and Comparative Examples A1 and A2. A fixed-bed reactor was provided by filling a reaction tube (inner diameter of 10 mm, length of 500 mm) made of stainless steel with 15 mL of a catalyst, and the reactor was immersed in a molten salt bath at 360°C. Thereafter, nitrogen gas was allowed to flow in the reactor at the rate of 62 mL/min for 30 minutes, and a reaction gas consisting of vapor of 80 mass% glycerin aqueous solution and nitrogen gas (reaction gas composition: 27 mol% of glycerin, 34 mol% of water, and 39 mol% of nitrogen) was allowed to flow in the reactor at the rate (GHSV) of 640 $hr^{-1}$ for 3 hours. For 30 minutes of from 2.5 hours to 3.0 hours from introducing the reaction gas into the reactor, effluent gas from the reactor was condensed in acetonitrile by cooling to recover. Hereinafter, "the cooled and absorbed substance of the recovered effluent gas" may be referred to as an "effluent".

A part of the effluent was collected and the qualitative and quantitative analysis of the effluent was carried out using a gas chromatography (GC) equipped with FID as a detector. In the quantitative analysis by the GC, an internal reference method was employed. As the result of the qualitative analysis by the GC, by-products such as propionaldehyde and the like were detected along with glycerin and acrolein. From the result of the quantitative analysis, conversion rate of glycerin (GLY conversion rate), selectivity of acrolein (ACR selectivity), selectivity of propionaldehyde (PALD selectivity), and selectivity of 1-hydroxyacetone (HDAC) were calculated. Calculating formulas of them were as follows:

$$\text{GLY conversion rate} = (1 - (\text{molar quantity of glycerin in the effluent}) / (\text{molar quantity of glycerin fed into the reactor for 30 minutes}) \times 100;$$

$$\text{ACR selectivity} = (\text{molar quantity of acrolein}) / (\text{molar quantity of glycerin fed into the reactor for 30 minutes}) \times 100 / \text{GLY conversion rate} \times 100;$$

$$\text{PALD selectivity} = (\text{molar quantity of propionaldehyde}) / (\text{molar quantity of glycerin fed into the reactor for 30 minutes}) \times 100 / \text{GLY conversion rate} \times 100;$$

$$\text{HDAC selectivity} = (\text{molar quantity of 1-hydroxyacetone}) / (\text{molar quantity of glycerin fed into the reactor for 30 minutes}) \times 100 / \text{GLY conversion rate} \times 100.$$

The molar ratios P/B of the respective catalysts in Examples A1 to A4 and Comparative Examples A1, A2 and the reaction results in the acrolein production using the respective catalysts are shown in Table 1. The molar ratios of the catalyst component are values calculated from blending amounts of raw materials used in the preparation of the boron phosphate. As shown in Table 1, the catalyst comprising boron phosphate wherein the molar ratio P/B of phosphorus (P) to boron (B) was 1.0 or more increased the selectivity of acrolein (ACR), comparing the results of Examples A1 to A4 and Comparative Example A2 with the result of Comparative Example A1. Meanwhile, comparing the results of Examples A1 to A4 with Comparative Examples A1 and A2, the catalyst having the molar ratio P/B more than 1.0 decreased the selectivity of propionaldehyde (PALD). Thus, when the catalyst having the molar ratio P/B more than 1.0 was used, acrolein could be produced in high yield while decreasing the production of by-products.

[Table 1]

| | Catalyst Preparing Method | Molar Ratio of Catalyst Component | | GLY Conv. Rate (%) | Selectivity (%) | | |
|---|---|---|---|---|---|---|---|
| | | P | B | | ACR | PALD | HDAC |
| Comp. Ex. A1 | Condensation Method | 0.83 | 1.0 | 100 | 67.9 | 0.80 | 9.6 |
| Comp. Ex. A2 | | 1.0 | 1.0 | 100 | 72.9 | 0.88 | 10.7 |
| Example A1 | | 1.05 | 1.0 | 100 | 73.8 | 0.68 | 10.4 |
| Example A2 | | 1.1 | 1.0 | 100 | 74.6 | 0.67 | 10.3 |
| Example A3 | | 1.2 | 1.0 | 100 | 71.6 | 0.64 | 10.1 |
| Example A4 | | 1.5 | 1.0 | 100 | 72.5 | 0.65 | 10.9 |

(1-3) Examples of acrolein production (long-term performance; catalyst lifetime)

Example A5

Acrolein was produced according to the method described in the above section (1-2), using the catalyst obtained in the above Example A1 as the catalyst for glycerin dehydration. However, the reaction gas was allowed to flow in the reactor for up to 48 hours. During this time, for 30 minutes before setup durations described in Table 2 from introducing the reaction gas into the reactor, effluent gas from the reactor was condensed in acetonitrile by cooling to recover and the effluent obtained at each of the setup durations was subjected to the qualitative and quantitative analysis. In Example A5, it was possible to produce acrolein for 48 hours without interruption.

Example A6

Acrolein was produced in the same manner as in Example A5, except using the catalyst obtained in the above Example A2 as the catalyst for glycerin dehydration. In Example A6, it was possible to produce acrolein for 48 hours without interruption.

Example A7

Acrolein was produced in the same manner as in Example A5, except using the catalyst obtained in the above Example A4 as the catalyst for glycerin dehydration. In Example A7, it was possible to produce acrolein for 48 hours without interruption.

Comparative Example A3

Acrolein was produced in the same manner as in Example A5, except using the catalyst obtained in the above Comparative Example A1 as the catalyst for glycerin dehydration. In Comparative Example A3, the pressure loss was drastically increased after 4.7 hours passage of the reaction time, resulting in being unable to producing acrolein any further.

Comparative Example A4

Acrolein was produced in the same manner as in Example A5, except using the catalyst obtained in the above Comparative Example A2 as the catalyst for glycerin dehydration, to evaluate the performance. In Comparative Example A4, the pressure loss gradually increased as the reaction time goes by, and the production of acrolein became impossible after 20 hours passage of the reaction time.

Comparison of results of Examples A5 to A7 and Comparative Examples A3, A4

The results of Examples A5 to A7 and Comparative Examples A3, A4 are shown in Table 2. As shown in Table 2, comparing the results of Examples A5 to A7 with the results of Comparative Examples A3 and A4, the catalyst comprising boron phosphate and having the molar ratio P/B more than 1.0 lengthened the reaction continuable time more than twice to the catalyst comprising boron phosphate having the molar ratio P/B of 1.0 or less. By using the catalyst having the molar ratio P/B more than 1.0, acrolein could be produced stably for a long period.

[Table 2]

| | Molar Ratio of Cat Comp. | | Reaction Time (h) | GLYConv. Rate (%) | Selectivity (%) | | |
|---|---|---|---|---|---|---|---|
| | P | B | | | ACR | PALD | HDAC |
| Comparative Example A3 | 0.83 | 1.0 | 1 | 100 | 70.6 | 0.6 | 8.3 |
| | | | 3 | 100 | 73.7 | 0.6 | 11.7 |
| | | | 4.5 | 100 | 72.9 | 0.9 | 12.8 |
| | | | Reaction was stopped at 4.7 hrs. due to pressure loss increase | | | | |
| Comparative Example A4 | 1.0 | 1.0 | 1 | 100 | 71.1 | 0.8 | 9.0 |
| | | | 5 | 100 | 69.2 | 0.9 | 9.2 |
| | | | 7 | 100 | 68.8 | 1.0 | 8.9 |
| | | | 12 | 100 | 72.8 | 0.9 | 9.4 |
| | | | 18 | 100 | 72.3 | 1.0 | 9.1 |
| | | | Reaction was stopped at 20 hrs. due to pressure loss increase | | | | |
| Example A5 | 1.05 | 1.0 | 2 | 100 | 72.6 | 0.5 | 11.4 |
| | | | 2.5 | 100 | 73.9 | 0.5 | 14.8 |
| | | | 4.5 | 100 | 73.7 | 0.6 | 15.0 |
| | | | 5 | 100 | 76.2 | 0.6 | 14.6 |
| | | | 7 | 100 | 76.0 | 0.6 | 15.2 |
| | | | 24 | 100 | 75.3 | 0.6 | 14.4 |
| | | | 26.4 | 100 | 74.5 | 0.8 | 15.0 |
| | | | 30 | 100 | 67.8 | 0.8 | 14.4 |
| | | | 47.5 | 100 | 66.1 | 0.9 | 14.8 |
| | | | 48 | 100 | 65.3 | 1.2 | 15.2 |

(continued)

| | Molar Ratio of Cat Comp. | | Reaction Time (h) | GLYConv. Rate (%) | Selectivity (%) | | |
|---|---|---|---|---|---|---|---|
| | P | B | | | ACR | PALD | HDAC |
| Example A6 | 1.1 | 1.0 | 2 | 100 | 72.6 | 0.2 | 11.4 |
| | | | 2.5 | 100 | 78.9 | 0.2 | 14.8 |
| | | | 4.5 | 100 | 73.7 | 0.2 | 15.0 |
| | | | 5 | 100 | 76.2 | 0.3 | 14.6 |
| | | | 7 | 100 | 77.0 | 0.3 | 15.2 |
| | | | 24 | 100 | 71.5 | 0.8 | 15.0 |
| | | | 26.4 | 100 | 66.8 | 1.0 | 14.4 |
| | | | 30 | 100 | 70.1 | 0.9 | 14.8 |
| | | | 47.5 | 100 | 68.2 | 1.1 | 15.2 |
| | | | 48 | 100 | 66.9 | 1.2 | 15.1 |
| Example A7 | 1.5 | 1.0 | 2 | 100 | 74.6 | 0.5 | 15.7 |
| | | | 2.5 | 100 | 75.0 | 0.5 | 15.9 |
| | | | 5 | 100 | 73.9 | 0.7 | 16.2 |
| | | | 7 | 100 | 75.2 | 0.6 | 16.1 |
| | | | 24 | 100 | 75.3 | 0.6 | 16.9 |
| | | | 27.5 | 100 | 75.4 | 0.8 | 15.2 |
| | | | 29.5 | 100 | 73.2 | 0.7 | 16.2 |
| | | | 30 | 100 | 68.0 | 0.8 | 15.8 |
| | | | 47.5 | 100 | 68.0 | 0.9 | 16.2 |
| | | | 48 | 100 | 66.2 | 1.1 | 15.8 |

(1-4) Example of acrylic acid production

Acrylic acid was produced from glycerin by the following steps.

(i) First reaction step: Glycerin was dehydrated to obtain a composition containing propionaldehyde and acrolein.
(ii) First purification step: The reaction product obtained in the step (i) was purified to obtain a composition containing propionaldehyde and acrolein which is able to be supplied to a second reaction.
(iii) Second reaction step: The composition obtained in the step (ii) was oxidized to obtain a reaction product containing crude acrylic acid.
(iv) Second purification step: The reaction product obtained in the step (iii) was - distilled to give crude acrylic acid and the crude acrylic acid was purified by crystallization to obtain acrylic acid.

The above respective steps are explained below in sequence.

(i) First reaction step

As a catalyst used for the first reaction (a catalyst for a first reaction), the catalyst obtained in Example A2 was used. 50 mL of this catalyst was put in a reaction tube (inner diameter of 25 mm, length of 500 mm) made of stainless steel, thereby preparing a fixed-bed reactor, and the reactor was installed in a niter bath at 360°C. A condenser was installed at an outlet of the reactor, and cooling water at about 4°C was introduced thereinto. The pressure of the reaction system was reduced to 62 kPa by a vacuum pump, while adjusting the pressure using a constant vacuum apparatus. Then, a glycerin-containing gas was introduced into the reactor. As the glycerin-containing gas, a mixed gas consisting of 44 volume% of glycerin and 56 volume% of water was introduced at a space velocity (GHSV) of 420 hr$^{-1}$. The glycerin-containing gas was fed for 20 hours from the start, and the gas flowing out of the reactor was entirely condensed by the condenser and recovered in a receiver cooled by an ice bath. The weight of the recovered reaction product was 939 g, which corresponded to 99 mass% of the supplied raw material. The obtained reaction product was quantitatively analyzed by a gas chromatograph, and as a result, it was found that the reaction product contained 36 mass% of acrolein, 0.3 mass% of propionaldehyde, 6.6 mass% of 1-hydroxyacetone, 45 mass% of water and 15 mass% of heavy components.

(ii) First purification step

The reaction product obtained in the step (i) was supplied at 0.12 kg/h to a thin film distiller, which was operated in the conditions of normal pressure, 85°C of wall temperature on which a liquid film was formed, and 300 rpm of blade rotation speed. A distillate containing 42 mass% of acrolein, 0.6 mass% of propionaldehyde, 4 mass% of 1-hydroxyacetone and 51 mass% of water was obtained at 0.11 kg/h from the top of the distiller.

(iii) Second reaction step

An oxidation catalyst used for the second reaction (a catalyst for a second reaction) was prepared as follows. Into 2500 mL of water which was stirred while heating, 350 g of ammonium paramolybdate, 116 g of ammonium metavanadate, and 44.6 g of ammonium paratungstate were dissolved, and then, 1.5 g of vanadium trioxide was further added thereto. Separately, into 750 mL of water which was stirred while heating, 87.8 g of copper nitrate was dissolved, and then, 1.2 g of copper (I) oxide and 29 g of antimony trioxide were added thereto. Thus obtained two solutions were mixed, 1000 mL of spherical $\alpha$-alumina having a diameter of 3 mm to 5 mm as a carrier was fed thereto, and then, the resultant mixture was evaporated to dryness while stirring to obtain a catalyst precursor. The catalyst precursor was calcinated at 400°C for 6 hours to give the oxidation catalyst for producing acrylic acid. The supported metal element composition of the oxidation catalyst for producing acrylic acid was $Mo_{12}V_{6.1}W_1Cu_{2.3}Sb_{1.2}$.

A fixed-bed oxidation reactor was provided by filling a reaction tube (inner diameter of 25 mm, length of 500 mm) made of stainless steel with 50 mL of the oxidation catalyst of the above, and the reactor was installed in a niter bath at 260°C. Then, the composition obtained in the step (ii) was supplied to the reactor. A condenser was installed at an outlet of the reactor, and cooling water at about 15°C was introduced thereinto. A mixed gas consisting of 6.5 volume% of acrolein, 0.06 volume% of propionaldehyde, 0.45 volume% of 1-hydroxyacetone, 6 volume% of oxygen, 13 volume% of water and 74 volume% of nitrogen was introduced as a propionaldehyde-containing gas at a space velocity (GHSV) of 1900 hr$^{-1}$. The composition containing propionaldehyde and acrolein was supplied for 22 hours from the start of the reaction, and the gas flown out of the reactor was condensed by the condenser and recovered in a receiver cooled by an ice bath and in a cold trap subsequently installed. The weight of the recovered reaction product which contains crude acrylic acid was 614 g, which corresponded to 95 mass% of the supplied raw material. Based on quantitatively analysis of gas chromatograph, it was found that the reaction product contained 62 mass% of acrylic acid, 0.01 mass% of propionic acid, 1 mass% of formic acid, 3 mass% of acetic acid and 34 mass% of water. In the production of acrylic acid using glycerin as a raw material, it was found that the amounts of byproducts of organic acids such as propionic acid and formic acid were increased compared with the conventional process for producing acrylic acid using propylene as a raw material.

(iv) Second purification step

The reaction product obtained in the step (iii) was supplied at 0.2 kg/h to the fifth stage of a distillation column having 10 stages and continuously distilled under the conditions of a refluxing ratio of 1 and a distillation amount from the top of the column of 0.070 kg/h. As a result, crude acrylic acid having composition of 88.1 mass% of acrylic acid, 0.01 mass% of propionic acid, 2.3 mass% of acetic acid, 0.04 mass% of formic acid and 9.5 mass% of water was obtained at 0.130 kg/h from the bottom of the column. Crystallization operation was conducted by cooling the crude acrylic acid as a mother liquid to a temperature range of -5.8°C to room temperature (about 15°C) to form a crystal, keeping at the same temperature, and then, separating the crystal from the liquid by suction filtration. The separated crystal was melted, and a portion of the crystal was sampled for analysis and the rest thereof was processed by the crystallization operation, in which the rest was cooled as a mother liquid to a temperature range of 4.6°C to room temperature (about 15°C) to form a crystal, kept at the same temperature, and then, the crystal was separated from the liquid by suction filtration. According to the crystallization operations repeated two times in total, acrylic acid with a purity of 99.9 mass% or higher was obtained finally. The results are shown in Table 3. The content of propionic acid was below a detection limit (1 ppm).

[Table 3]

|  | Mother liquid | First crystallization | Second crystallization |
|---|---|---|---|
| Acrylic acid (mass%) | 88.1 | 98.8 | 99.9 |
| Propionic acid (mass%) | 0.01 | N.D. | N.D. |
| Acetic acid (mass%) | 2.3 | 0.3 | 0.02 |
| Water (mass%) | 9.5 | 0.9 | 0.08 |
| Temperature (°C) | - | -5.8 | 4.6 |

N.D. means a value below the detection limit (1 ppm by mass).

(1-5) Example of production of a water-absorbent resin

Acrylic acid containing acetic acid and propionic acid in a total amount of 200 ppm by mass, which was obtained in the second crystallization in Example of acrylic acid production, was mixed with a polymerization inhibitor to give acrylic acid containing 60 ppm by mass of the polymerization inhibitor. The above acrylic acid was added to NaOH aqueous solution, which had been separately prepared from sodium hydroxide containing 0.2 ppm by mass of iron, under cooling condition (solution temperature 35°C) to be neutralized at 75 mol%. The contents of iron in the acrylic acid and the water were below a detection limit, and thus, the iron content in a monomer was about 0.07 ppm by mass based on the calculated value.

Into the obtained 35 mass% sodium acrylate aqueous solution, which has a neutralization ratio of 75 mol%, 0.05 mol% (relative to the sodium acrylate aqueous solution) of polyethylene glycol diacrylate as an internal crosslinking agent was dissolved to obtain a monomeric component. 350 g of the monomeric component was fed to a cylindrical vessel with a volume of 1 L, and nitrogen was blown at 2 L/minute into the vessel for 20 minutes to expel the air. Then, an aqueous solution containing 0.12 g/mol (relative to the monomeric component) of sodium persulfate and 0.005 g/mol (relative to the monomeric component) of L-ascorbic acid were added thereto while being stirred by a stirrer to start polymerization. After the start of the polymerization, stirring was stopped and static aqueous solution polymerization was conducted. The temperature of the monomeric component reached peak polymerization temperature of 108°C after about 15 minutes (polymerization peak time), and then, polymerization was proceeded for 30 minutes. Subsequently, the resulting polymer material was taken out of the cylindrical vessel to obtain a hydrated gel-like crosslinked polymer. The obtained hydrated gel-like crosslinked polymer was segmented at 45°C by a meat chopper (hole diameter 8 mm) and dried at 170°C for 20 minutes by a hot-air drier. Further, the dried polymer (solid matter: about 95%) was pulverized by a roll mill and classified by a JIS standard sieve into those having a particle diameter of 300 $\mu$m to 600 $\mu$m, thereby obtaining a polyacrylic acid water-absorbent resin (neutralization ratio 75%).

The obtained acrylic acid had the comparable polymerization property to acrylic acid obtained by the process of producing acrylic acid from propylene, and the obtained hydrophilic resin was free from odor and had the same physical properties as a conventional one.

(2) Catalyst comprising a rare-earth metal phosphate

(2-1) Preparation of catalyst

Example B1 (Preparation of catalyst by a sol-gel method)

Into the aqueous solution obtained by adding 1800 g of distilled water to 461.14 g of $Nd(NO_3)_3$ aqueous solution (available from Shin-Etsu Chemical Co., Ltd.; concentration in terms of $Nd_2O_3$: 221 g/kg) under stirring, 187.58 g of 28 wt% ammonia water was added dropwise at a constant rate over 3 hours using a pump for high-performance liquid chromatography ("L7110" available from Hitachi, Ltd.). The solution after the dropwise addition was aged by being left for 15 hours under stirring to obtain a solution containing neodymium hydroxide. Into this solution under stirring, 78.22 g of phosphoric acid aqueous solution ($H_3PO_4$ concentration: 85 mass%) was added dropwise at a constant rate over 3 hours using a pump for high-performance liquid chromatography. The solution after the dropwise addition was left for 15 hours under stirring to obtain a sol- or gel-like material containing phosphate and neodymium (a rare-earth metal). The sol- or gel-like material was dewatered under the conditions of 0.005 MPa and 60°C, followed by drying under air atmosphere at 120°C for 10 hours. Thus obtained dried product was placed in nitrogen atmosphere at 450°C for 10 hours (a pre-heat treatment) for the purpose of separating and decomposing ammonium components which is assumed to exist in the dried product, followed by calcinating under air atmosphere at 1000°C for 5 hours to give a calcinated product. The calcinated product was pulverized and sieved by using sieves with opening apertures of 0.7 mm or 2.0 mm, thereby obtaining a classified calcinated product with the size of 0.7 mm to 2.0 mm as a catalyst. The obtained catalyst was a catalyst for glycerin dehydration comprising neodymium phosphate of a rare-earth metal phosphate.

Examples B2, B3 and Comparative Examples B1 to B3 (Preparation of catalyst by a sol-gel method)

Catalysts were prepared in the same manner as in Example B1, except that the used amounts of $Nd(NO_3)_3$ aqueous solution, ammonia water and phosphoric acid aqueous solution were changed as described in Table 4.

[Table 4]

| | Catalyst Preparing Method | Molar Ratio of Catalyst Component | | Amounts of Raw Materials (g) | | |
|---|---|---|---|---|---|---|
| | | P | R(Nd) | $Nd(NO_3)_3$ aq. solution | $H_3PO_4$ aq. solution | Ammonia Water |
| Comp. Ex. B1 | Sol-gel Method | 0.8 | 1.0 | 505.39 | 62.34 | 205.58 |
| Comp. Ex. B2 | | 0.9 | 1.0 | 489.73 | 67.96 | 199.21 |
| Comp. Ex. B3 | | 1.0 | 1.0 | 475.01 | 73.25 | 193.22 |
| Example B1 | | 1.1 | 1.0 | 461.14 | 78.22 | 187.58 |
| Example B2 | | 1.2 | 1.0 | 448.07 | 82.91 | 182.26 |
| Example B3 | | 1.5 | 1.0 | 412.94 | 95.51 | 167.97 |

Example B4 (Preparation of catalyst by a kneading method)

69.34 g of finely-pulverized neodymium oxide (available from Kanto Chemical Co., Inc.) and 57.61 g of diammonium hydrogenphosphate were weighed and fed to a mortar in sequence, and then, mixed for 30 minutes while being pulverized by a pestle. The obtained mixture was dried under air atmosphere at 120°C for 24 hours. Thus obtained dried product was placed in air atmosphere at 450°C for 10 hours (a pre-heat treatment) for the purpose of separating and decomposing ammonium components which is assumed to exist in the dried product, followed by calcinating under air atmosphere at 1000°C for 5 hours to give a calcinated product. The calcinated product was sieved by using sieves with opening apertures of 0.7 mm or 2.0 mm, thereby obtaining a classified calcinated product with the size of 0.7 mm to 2.0 mm as a catalyst. The obtained catalyst was a catalyst for glycerin dehydration comprising neodymium phosphate of a rare-earth metal phosphate.

Examples B5, B6 (Preparation of catalyst by a kneading method)

Catalysts were prepared in the same manner as in Example B4, except that the used amounts of neodymium oxide and diammonium hydrogenphosphate were changed as described in Table 5.

[Table 5]

| | Catalyst Preparing Method | Molar Ratio of Catalyst Component | | Amounts of Raw Materials (g) | |
|---|---|---|---|---|---|
| | | P | R(Nd) | $Nd_2O_3$ | $(NH_4)_2HPO_4$ |
| Example B4 | | 1.05 | 1.0 | 69.34 | 57.61 |
| Example B5 | Kneading Method | 1.1 | 1.0 | 68.34 | 59.49 |
| Example B6 | | 1.2 | 1.0 | 66.42 | 63.08 |

Example B7 (Preparation of catalyst by a sol-gel method)

A catalyst was prepared in the same manner as in Example B 1, except using the aqueous solution obtained by adding 820.0 g of distilled water to 200.8 g of yttrium nitrate (available from Kishida Chemical Co., Ltd.) in place of the aqueous solution obtained by adding 1800 g of distilled water to 461.14 g of $Nd(NO_3)_3$ aqueous solution, and changing the used amounts of ammonia water and phosphoric acid aqueous solution as described in Table 6. The obtained catalyst was a catalyst for glycerin dehydration comprising yttrium phosphate of a rare-earth metal phosphate.

Examples B8, B9 and Comparative Example B4 (Preparation of catalyst by a sol-gel method)

Catalyst were prepared in the same manner as in Example B7, except that the used amounts of yttrium nitrate, distilled water, ammonia water and phosphoric acid aqueous solution were changed as described in Table 6. In Comparative Example B4, calcination was conducted at 1100°C.

[Table 6]

| | Catalyst Preparing Method | Molar Ratio of Amounts of Raw Materials (g) Cat. Component | | | | | |
|---|---|---|---|---|---|---|---|
| | | P | R(Y) | $Y(NO_3)_3 .6H_2O$ | Distilled Water | $H_3PO_4$ aq. Sol. | Ammonia Water |
| Comp. Ex. B4 | | 1.0 | 1.0 | 208.5 | 812.0 | 62.7 | 165.4 |
| Example B7 | Sol-gel Method | 1.1 | 1.0 | 200.8 | 820.0 | 66.4 | 159.3 |
| Example B8 | | 1.2 | 1.0 | 193.6 | 826.6 | 69.8 | 153.5 |
| Example B9 | | 1.5 | 1.0 | 174.8 | 844.8 | 78.8 | 138.6 |

Examples B10 to B12 and Comparative Example B5 (Preparation of catalyst by a sol-gel method)

Catalyst were prepared in the same manner as in Example B7, except that cerium nitrate was used by the amount described in Table 7 in place of yttrium nitrate and the used amounts of distilled water, ammonia water and phosphoric acid aqueous solution were changed as described in Table 7. The obtained catalysts were catalysts for glycerin dehydration comprising cerium phosphate of a rare-earth metal phosphate.

[Table 7]

| | Catalyst Preparing Method | Molar Ratio of Cat. Component | | Amounts of Raw Materials (g) | | | |
|---|---|---|---|---|---|---|---|
| | | P | R(Ce) | $Ce(NO_3)_3 \cdot 6H_2O$ | Distilled Water | $H_3PO_4$ aq. Sol. | Ammonia Water |
| Comp. Ex. B5 | Sol-gel Method | 1.0 | 1.0 | 184.9 | 924.6 | 49.0 | 129.4 |
| Example B10 | | 1.1 | 1.0 | 179.5 | 927.2 | 52.4 | 125.6 |
| Example B11 | | 1.2 | 1.0 | 174.4 | 929.7 | 55.5 | 122.0 |
| Example B12 | | 1.5 | 1.0 | 160.6 | 936.3 | 63.9 | 112.4 |

(2-2) Examples of acrolein production (initial performance)

Glycerin was dehydrated to produce acrolein according to the method described in the above section (1-2), using the catalyst obtained in each of the above Examples B1 to B12 and Comparative Examples B1 to B5, and the conversion rate of glycerin (GLY conversion rate), the selectivity of acrolein (ACR selectivity), and the selectivity of propionaldehyde (PALD selectivity) were calculated. The results are shown in Table 8, and it was found that in the cases of using the catalyst comprising neodymium phosphate as a rare-earth metal phosphate wherein the molar ratio P/R of phosphorus (P) to the rare-earth metal (R) was more than 1.0 (Examples B1 to B6), the selectivity of propionaldehyde (PALD) could be kept low while maintaining the selectivity of acrolein (ACR) relatively high. In the catalyst having the molar ratio P/R more than 1.0 (Examples B1 to B6), the selectivity of propionaldehyde (PALD) fell to less than about one-fourth of that in the catalyst having the molar ratio P/R of 1.0 or less (Comparative Examples B1 to B3). Also, in the cases of using the catalyst comprising yttrium phosphate or cerium phosphate as a rare-earth metal phosphate wherein the molar ratio P/R of phosphorus (P) to the rare-earth metal (R) was more than 1.0 (Example B7 to B12), the selectivity of propionaldehyde (PALD) could be kept low while maintaining the selectivity of acrolein (ACR) relatively high. The catalyst having the molar ratio P/B more than 1.0 (Example B7 to B12) decreased the selectivity of propionaldehyde (PALD) as compared with the catalyst having the molar ratio P/R of 1.0 or less (Comparative Examples B4 and B5).

[Table 8]

| | Catalyst Preparing Method | Rare-earth Metal (R) | Molar Ratio of Cat. Component | | GLY Conv. Rate (%) | Selectivity (%) | |
|---|---|---|---|---|---|---|---|
| | | | P | R | | ACR | PALD |
| Comp. Ex. B1 | Sol-gel Method | Nd | 0.8 | 1.0 | 100 | 41.9 | 3.7 |
| Comp. Ex. B2 | | Nd | 0.9 | 1.0 | 100 | 60.5 | 4.4 |
| Comp. Ex. B3 | | Nd | 1.0 | 1.0 | 100 | 77.9 | 4.2 |
| Example B1 | | Nd | 1.1 | 1.0 | 100 | 68.9 | 1.1 |
| Example B2 | | Nd | 1.2 | 1.0 | 100 | 68.5 | 1.0 |
| Example B3 | | Nd | 1.5 | 1.0 | 100 | 69.6 | 0.8 |

(continued)

| | Catalyst Preparing Method | Rare-earth Metal (R) | Molar Ratio of Cat. Component | | GLY Conv. Rate (%) | Selectivity (%) | |
|---|---|---|---|---|---|---|---|
| | | | P | R | | ACR | PALD |
| Example B4 | Kneading Method | Nd | 1.05 | 1.0 | 100 | 65.9 | 0.6 |
| Example B5 | | Nd | 1.1 | 1.0 | 100 | 64.2 | 0.3 |
| Example B6 | | Nd | 1.2 | 1.0 | 100 | 69.9 | 0.3 |
| Comp. Ex. B4 | Sol-gel Method | Y | 1.0 | 1.0 | 100 | 66.1 | 1.9 |
| Example B7 | | Y | 1.1 | 1.0 | 100 | 68.9 | 1.3 |
| Example B8 | | Y | 1.2 | 1.0 | 100 | 68.9 | 1.4 |
| Example B9 | | Y | 1.5 | 1.0 | 100 | 63.6 | 1.2 |
| Comp. Ex. B5 | Sol-gel Method | Ce | 1.0 | 1.0 | 100 | 68.6 | 1.7 |
| Example B10 | | Ce | 1.1 | 1.0 | 100 | 64.6 | 1.2 |
| Example B 11 | | Ce | 1.2 | 1.0 | 100 | 64.2 | 1.1 |
| Example B12 | | Ce | 1.5 | 1.0 | 100 | 63.7 | 1.0 |

(2-3) Examples of acrolein production (long-term performance; catalyst lifetime)

Example B 13

Acrolein was produced according to the method described in the above section (2-2), using the catalyst obtained in the above Example B1 as the catalyst for glycerin dehydration. However, the reaction gas was allowed to flow in the reactor for 24 hours. During this time, for 30 minutes before the setup durations (3, 5, 7, 9, 18 or 24 hours) from introducing the reaction gas into the reactor, effluent gas from the reactor was condensed in acetonitrile by cooling to recover and the effluent obtained at each of the setup durations was subjected to the qualitative and quantitative analysis.

Example B14

Acrolein was produced in the same manner as in Example B13, except using the catalyst obtained in the above Example B2 as the catalyst for glycerin dehydration.

Example B 15

Acrolein was produced in the same manner as in Example B13, except using the catalyst obtained in the above Example B3 as the catalyst for glycerin dehydration.

Comparison of results of Examples B 13 to B 15

The results of Examples B13 to B15 are shown in Table 9. As shown in Table 9, in the cases of using the catalyst comprising a rare-earth metal phosphate wherein the molar ratio P/R of phosphorus (P) to the rare-earth metal (R) was more than 1.0 (Examples B13 to B15), the selectivity of propionaldehyde (PALD) could be kept low while maintaining the selectivity of acrolein (ACR) relatively high even after 24 hours from the start of the reaction. However, in the case of using the catalyst wherein the molar ratio P/R of phosphorus (P) to a rare-earth metal (R) was 1.5 or more (Example B15), the conversion rate of glycerin (GLY) began to decrease at the time of 9 hours duration from the start of the reaction, and it was found that the catalyst was inferior in respect of catalyst lifetime. On the other hand, in the cases of using the catalyst having the molar ratio P/R less than 1.5 (Examples B13 and B14), degradation of the catalyst performance was not observed even after 24 hours from the start of the reaction.

[Table 9]

| | Catalyst Preparing Method | Molar Ratio of Cat. Component | | Reaction Time (h) | GLY Conv. Rate (%) | Selectivity (%) | |
|---|---|---|---|---|---|---|---|
| | | P | R(Nd) | | | ACR | PALD |
| Example B13 | Sol-gel Method | 1.1 | 1.0 | 3 | 100 | 68.9 | 1.1 |
| | | | | 5 | 100 | 68.5 | 0.9 |
| | | | | 7 | 100 | 68.5 | 0.9 |
| | | | | 9 | 100 | 68.4 | 1.1 |
| | | | | 18 | 100 | 68.7 | 1.2 |
| | | | | 24 | 100 | 68.9 | 1.2 |
| Example B14 | Sol-gel Method | 1.2 | 1.0 | 3 | 100 | 68.5 | 1.0 |
| | | | | 5 | 100 | 69.1 | 1.0 |
| | | | | 7 | 100 | 68.5 | 1.0 |
| | | | | 9 | 100 | 67.2 | 1.0 |
| | | | | 18 | 100 | 68.3 | 1.2 |
| | | | | 24 | 100 | 69.1 | 1.2 |
| Example B15 | Sol-gel Method | 1.5 | 1.0 | 3 | 100 | 69.6 | 0.8 |
| | | | | 5 | 100 | 69.0 | 0.7 |
| | | | | 7 | 100 | 68.9 | 0.8 |
| | | | | 9 | 99.8 | 67.9 | 0.9 |
| | | | | 18 | 98.2 | 67.9 | 1.0 |
| | | | | 24 | 97.1 | 67.9 | 1.1 |

(2-4) Example of acrylic acid production

Acrylic acid was produced from glycerin according to the producing process described in the above section (1-4) except the following points.

(i) First reaction step

A catalyst obtained in Example B1 was used as a catalyst used for the first reaction (a catalyst for the first reaction), and the glycerin-containing gas was supplied for 24 hours. The weight of the recovered reaction product was 1088 g, which corresponded to 98 mass% of the supplied raw material. The obtained reaction product contained 34 mass% of acrolein, 0.4 mass% of propionaldehyde, 8.0 mass% of 1-hydroxyacetone, 45 mass% of water and 12.6 mass% of heavy components.

(ii) First purification step

The reaction product obtained in the step (i) was supplied at 0.48 kg/h to the fifth stage of an Oldershow continuous distillation column having 10 stages under the conditions of a refluxing ratio of 2 and the bottom temperature of 96°C. A distillate containing 97 mass% of acrolein, 0.3 mass% of propionaldehyde and 3 mass% of water was obtained at 0.16 kg/h from the top of the distiller.

(iii) Second reaction step

As the propionaldehyde-containing gas, a mixed gas consisting of 6.5 volume% of acrolein, 0.03 volume% of propionaldehyde, 6 volume% of oxygen, 13 volume% of water and 74 volume% of nitrogen was introduced at a space velocity (GHSV) of 1600 hr$^{-1}$. The weight of the recovered reaction product was 503 g, which corresponded to 98 mass% of the supplied raw material. The reaction product contained 65.6 mass% of acrylic acid, 0.16 mass% of propionic acid, 0.8 mass% of formic acid, 1.04 mass% of acetic acid and 32.4 mass% of water.

(iv) Second purification step

Crude acrylic acid having composition of 88.4 mass% of acrylic acid, 0.24 mass% of propionic acid, 1.02 mass% of acetic acid, 0.05 mass% of formic acid and 10.3 mass% of water was obtained at 0.140 kg/h from the bottom of the distillation column. In the crystallization operation, the mother liquid was cooled to a temperature range of -5.5°C to room temperature (about 15°C) to form a crystal at the first crystallization, and the mother liquid was cooled to a temperature range of 5.0°C to room temperature (about 15°C) to form a crystal at the second crystallization. The finally obtained acrylic acid had a purity of 99.9 mass% or higher, and the content of propionic acid was below a detection limit (1 ppm).

[Table 10]

|  | Mother liquid | First crystallization | Second crystallization |
|---|---|---|---|
| Acrylic acid (mass%) | 88.4 | 98.8 | 99.9 |
| Propionic acid (mass%) | 0.24 | N.D. | N.D. |
| Acetic acid (mass%) | 1.02 | 0.2 | 0.02 |
| Water (mass%) | 10.3 | 1.0 | 0.08 |
| Temperature (°C) | - | -5.5 | 5.0 |
| N.D. means a value below the detection limit (1 ppm by mass). | | | |

(2-5) Example of production of a water-absorbent resin

A water-absorbent resin was produced from acrylic acid according to the producing process described in the above section (1-5). The obtained acrylic acid had the comparable polymerization property to acrylic acid obtained by the process of producing acrylic acid from propylene, and the obtained hydrophilic resin was free from odor and had the same physical properties as a conventional one.

(3) Catalyst comprising boron phosphate and a metal element

(3-1) Preparation of catalyst

Example C1 (Preparation of catalyst by a condensation method)

Into a solution obtained by dissolving 198.55 g of boric acid in 1587.5 g of distilled water, 1.88 g of cesium nitrate was added as a metal source compound and well-stirred to give a uniform solution. Separately, 212.49 g of distilled water was added slowly to 303.56 g of 85% phosphoric acid aqueous solution to obtain 50% phosphoric acid aqueous solution. This 50% phosphoric acid aqueous solution was added to the above solution to give a mixed solution. The obtained mixed solution was heated to reflux at 90°C for 2 hours while stirring, thereby obtaining a clear, colorless catalyst precursor solution. The catalyst precursor solution was dewatered to condense on a hot-water bath of 60°C under reduced pressure condition of 0.005 MPa by using an evaporator. Thus obtained condensed matter was dried at 120°C under airflow for 24 hours to obtain a solid matter. The solid matter was calcinated at 1000°C under airflow for 5 hours. Thus obtained calcinated product was sieved by using sieves with opening apertures of 0.7 mm or 2.0 mm, thereby obtaining a classified calcinated product with the size of 0.7 mm to 2.0 mm as a catalyst. The obtained catalyst was a catalyst for glycerin dehydration comprising boron phosphate and cesium as a metal element.

Examples C2 to C14 and Comparative Examples C1 to C3 (Preparation of catalyst by a condensation method)

Catalysts were prepared in the same manner as in Example C1, except that the used amounts of boric acid, cesium nitrate, 85% phosphoric acid aqueous solution and distilled water were changed as described in Table 11.

[Table 11]

| | Catalyst Preparing Method | Molar Ratio of Catalyst Component | | | | Amounts of Raw Materials (g) | | | | |
|---|---|---|---|---|---|---|---|---|---|---|
| | | M | P | B | M/(P+B) | $CsNO_3$ | Boric Acid | Distilled Water | Phosphoric Acid aq. Sol. | |
| | | | | | | | | | 85% $H_3PO_4$ | Distilled Water |
| Comp. Ex. C1 | Condensation Method | - | 1.0 | 1.0 | 0 | - | 175.36 | 1571.1 | 326.96 | 228.87 |
| Comp. Ex. C2 | | 0.0001 | 1.0 | 1.0 | 0.000050 | 0.0553 | 175.34 | 1571.2 | 326.92 | 228.84 |
| Comp. Ex. C3 | | 0.0001 | 1.1 | 1.0 | 0.000048 | 0.0518 | 164.32 | 1564.1 | 337.00 | 235.90 |
| Example C1 | | 0.003 | 0.82 | 1.0 | 0.0016 | 1.88 | 198.55 | 1587.5 | 303.56 | 212.49 |
| Example C2 | | 0.01 | 1.0 | 1.0 | 0.0050 | 5.46 | 173.06 | 1574.1 | 322.66 | 225.87 |
| Example C3 | | 0.1 | 1.0 | 1.0 | 0.050 | 48.78 | 154.75 | 1598.0 | 288.53 | 201.97 |
| Example C4 | | 0.001 | 1.1 | 1.0 | 0.00048 | 0.52 | 164.13 | 1564.4 | 336.63 | 235.64 |
| Example C5 | | 0.005 | 1.1 | 1.0 | 0.0024 | 2.57 | 163.32 | 1565.5 | 334.96 | 234.47 |
| Example C6 | | 0.01 | 1.1 | 1.0 | 0.0048 | 5.12 | 162.31 | 1567.0 | 332.89 | 233.02 |
| Example C7 | | 0.05 | 1.1 | 1.0 | 0.024 | 24.38 | 154.68 | 1577.9 | 317.25 | 222.07 |
| Example C8 | | 0.1 | 1.1 | 1.0 | 0.048 | 46.05 | 146.10 | 1590.3 | 299.64 | 209.75 |
| Example C9 | | 0.5 | 1.1 | 1.0 | 0.24 | 159.47 | 108.18 | 1654.7 | 207.52 | 145.27 |
| Example C10 | | 0.01 | 1.2 | 1.0 | 0.0045 | 4.82 | 152.82 | 1560.7 | 341.92 | 239.34 |
| Example C11 | | 0.05 | 1.2 | 1.0 | 0.023 | 23.02 | 146.04 | 1571.3 | 326.75 | 228.72 |
| Example C12 | | 0.1 | 1.2 | 1.0 | 0.045 | 43.62 | 138.36 | 1583.3 | 309.58 | 216.70 |
| Example C13 | | 0.3 | 1.2 | 1.0 | 0.14 | 108.12 | 114.33 | 1620.9 | 255.81 | 179.06 |
| Example C14 | | 0.5 | 1.2 | 1.0 | 0.23 | 153.53 | 97.41 | 1647.4 | 217.95 | 152.57 |

Example C15 (Preparation of catalyst by a kneading method)

159.40 g of finely-pulverized boric acid, 25.12 g of cesium nitrate as a metal source compound, and 357.45 g of diammonium hydrogenphosphate were weighed and fed to a mortar in sequence, and then, mixed for 30 minutes while being pulverized by a pestle. The obtained mixture was dried under air atmosphere at 120°C for 24 hours. Thus obtained dried product was placed in air atmosphere at 450°C for 10 hours (a pre-heat treatment) for the purpose of separating and decomposing ammonium components which is assumed to exist in the dried product, followed by calcinating under air atmosphere at 1000°C for 5 hours to give a calcinated product. The calcinated product was sieved by using sieves with opening apertures of 0.7 mm or 2.0 mm, thereby obtaining a classified calcinated product with the size of 0.7 mm to 2.0 mm as a catalyst. The obtained catalyst was a catalyst for glycerin dehydration comprising boron phosphate and cesium as a metal element.

Examples C16 to C24 (Preparation of catalyst by a kneading method)

Catalysts were prepared in the same manner as in Example C 15, except that the used amounts of boric acid, cesium nitrate and diammonium hydrogenphosphate were changed as described in Table 12.

Examples C25 to C30 (Preparation of catalyst by a kneading method)

Catalysts were prepared in the same manner as in Example C15, except that the used amounts of boric acid and diammonium hydrogenphosphate were changed as described in Table 12 and cesium carbonate was used by the amount described in Table 12 in place of cesium nitrate.

[Table 12]

| | Catalyst Preparing Method | Molar Ratio of Catalyst Component | | | | Amounts of Raw Materials (g) | | | |
|---|---|---|---|---|---|---|---|---|---|
| | | M | P | B | M/(P+B) | Metal Source Compound | | Boric Acid | $(NH_4)_2HPO_4$ |
| Example C15 | Kneading Method | 0.05 | 1.05 | 1.0 | 0.024 | CsNO$_3$ | 25.12 | 159.40 | 357.45 |
| Example C16 | | 0.1 | 1.05 | 1.0 | 0.049 | | 47.38 | 150.30 | 337.04 |
| Example C17 | | 0.2 | 1.05 | 1.0 | 0.098 | | 85.05 | 134.90 | 302.51 |
| Example C18 | | 0.3 | 1.05 | 1.0 | 0.15 | | 115.71 | 122.36 | 274.39 |
| Example C19 | | 0.01 | 1.1 | 1.0 | 0.0048 | | 5.12 | 162.31 | 381.31 |
| Example C20 | | 0.05 | 1.1 | 1.0 | 0.024 | | 24.38 | 154.68 | 363.39 |
| Example C21 | | 0.1 | 1.1 | 1.0 | 0.048 | | 46.05 | 146.10 | 343.22 |
| Example C22 | | 0.2 | 1.1 | 1.0 | 0.095 | | 82.91 | 131.50 | 308.94 |
| Example C23 | | 0.3 | 1.1 | 1.0 | 0.14 | | 113.06 | 119.56 | 280.88 |
| Example C24 | | 0.5 | 1.1 | 1.0 | 0.24 | | 159.47 | 101.18 | 237.70 |
| Example C25 | | 0.01 | 1.1 | 1.0 | 0.0048 | Cs$_2$CO$_3$ | 4.28 | 162.31 | 381.31 |
| Example C26 | | 0.05 | 1.1 | 1.0 | 0.024 | | 20.38 | 154.68 | 363.39 |
| Example C27 | | 0.1 | 1.1 | 1.0 | 0.048 | | 38.49 | 146.10 | 343.22 |
| Example C28 | | 0.2 | 1.1 | 1.0 | 0.095 | | 69.29 | 131.50 | 308.94 |
| Example C29 | | 0.3 | 1.1 | 1.0 | 0.14 | | 94.50 | 119.56 | 280.88 |
| Example C30 | | 0.5 | 1.1 | 1.0 | 0.24 | | 133.29 | 101.18 | 237.70 |

Examples C31 to C40 (Preparation of catalyst by a kneading method)

Catalysts were prepared in the same manner as in Example C15, except that the used amounts of boric acid and diammonium hydrogenphosphate were changed as described in Table 13 and the compounds described in Table 13 were used by the amount described in Table 13 in place of cesium nitrate. The obtained catalyst were catalysts for glycerin dehydration comprising boron phosphate and a metal element.

[Table 13]

| Catalyst Preparing Method | Molar Ratio of Catalyst Component | | | | Amounts of Raw Materials (g) | | | |
|---|---|---|---|---|---|---|---|---|
| | | M | P | B | M/(P+B) | Metal Source Compound | Boric Acid | $(NH_4)_2HPO_4$ |
| Example C31 | Kneading Method | 0.1 | 1.1 | 1.0 | 0.048 | $LiNO_3$ | 18.08 | 162.19 | 381.02 |
| Example C32 | | 0.1 | 1.1 | 1.0 | 0.048 | $NaNO_3$ | 21.98 | 159.94 | 375.75 |
| Example C33 | | 0.03 | 1.1 | 1.0 | 0.014 | $AgNO_3$ | 13.14 | 159.43 | 374.53 |
| Example C34 | | 0.004 | 1.1 | 1.0 | 0.0019 | $Al(NO_3)_3 \cdot 9H_2O$ | 3.98 | 163.96 | 385.19 |
| Example C35 | | 0.02 | 1.1 | 1.0 | 0.0095 | $KNO_3$ | 5.33 | 163.14 | 386.19 |
| Example C36 | | 0.02 | 1.1 | 1.0 | 0.0095 | $RbNO_3$ | 7.71 | 161.82 | 383.07 |
| Example C37 | | 0.02 | 1.1 | 1.0 | 0.0095 | $Mg(NO_3)_2 \cdot 6H_2O$ | 13.67 | 163.33 | 386.65 |
| Example C38 | | 0.02 | 1.1 | 1.0 | 0.0095 | $Ca(NO_3)_2 \cdot 4H_2O$ | 12.62 | 162.88 | 385.58 |
| Example C39 | | 0.02 | 1.1 | 1.0 | 0.0095 | $Ba(NO_3)_2$ | 13.66 | 160.15 | 379.12 |
| Example C40 | | 0.02 | 1.1 | 1.0 | 0.0095 | $Ce(NO_3)_3 \cdot 6H_2O$ | 44.07 | 155.46 | 368.01 |

Example C41 (Preparation of catalyst by a kneading method)

144.58 g of finely-pulverized boric acid, 4.01 g of cerium oxide and 45.47 g of cesium nitrate as metal source compounds, and 339.99 g of diammonium hydrogenphosphate were weighed and fed to a mortar in sequence, and then, mixed for 30 minutes while being pulverized by a pestle. The obtained mixture was dried, pre-heated, calcinated and classified in the same manner as in Example C15, to prepare a catalyst. The obtained catalyst was a catalyst for glycerin dehydration comprising cerium and cesium as a metal elements in addition to boron phosphate.

Examples C42, C43

Catalysts were prepared in the same manner as in Example C41, except that the used amounts of boric acid, cerium oxide, cesium nitrate and diammonium hydrogenphosphate were changed as described in Table 14.

[Table 14]

| | Catalyst Preparing Method | Molar Ratio of Catalyst Component | | | | | Amounts of Raw Materials (g) | | | | | |
|---|---|---|---|---|---|---|---|---|---|---|---|---|
| | | $M_1$ (Ce) | $M_2$ (Cs) | P | B | M/(P+B) | Metal Source Compound | | | | Boric Acid | $(NH_4)_2 HPO_4$ |
| Example C41 | Kneading Method | 0.01 | 0.1 | 1.1 | 1.0 | 0.0524 | $CeO_2$ | 4.01 | $CsNO_3$ | 45.47 | 144.58 | 339.99 |
| Example C42 | | 0.05 | 0.1 | 1.1 | 1.0 | 0.0714 | | 19.05 | | 43.16 | 137.23 | 322.73 |
| Example C43 | | 0.1 | 0.1 | 1.1 | 1.0 | 0.0952 | | 35.82 | | 40.59 | 129.04 | 303.46 |

Example C44 (Preparation of supported catalyst by an impregnation method)

As a carrier to support a catalyst component, silica beads (a spherical silica carrier "Cariact Q50", available from Fuji Silysia Chemical Ltd.; particle diameter of from 0.85 mesh to 1.70 mesh; a carrier having uniform pores of average size of 50 nm) were used (hereinafter, this carrier is referred to as a "silica carrier"). Separately, into a solution obtained by dissolving 3.09 g of boric acid in 33.27 g of distilled water, 2.89 g of cesium nitrate was added as a metal source compound and well-stirred to give a mixed solution. 6.33 g of 85% phosphoric acid aqueous solution was added slowly to the obtained mixed solution, and the resultant was heated to 50°C while stirring, thereby obtaining a clear, colorless catalyst solution. The catalyst solution was added dropwise little by little onto 30 g of the silica carrier, which had been dried at 120°C for a day, and the resultant was left at room temperature for one hour, whereby the silica carrier was impregnated with the catalyst solution. The silica carrier impregnated with the catalyst solution was dried on an evaporating dish installed on a hot-water bath of 100°C to obtain a solid matter. The solid matter was dried at 120°C under airflow for 24 hours, and further calcined at 800°C in air atmosphere for 5 hours. Thus obtained calcined product was used as a catalyst. The obtained catalyst was a catalyst for glycerin dehydration in which boron phosphate and cesium as a metal element was supported on the silica carrier.

Examples C45 to C48 (Preparation of supported catalyst by an impregnation method)

Catalysts were prepared in the same manner as in Example C44, except that the used amounts of the silica carrier, boric acid, cesium nitrate, 85% phosphoric acid aqueous solution and distilled water were changed as described in Table 15.

Example C49 (Preparation of supported catalyst by an impregnation method)

As a carrier to support a catalyst component, silica having binary pores (a spherical silica carrier "NKS-13-2-1-7-2mm$\Phi$", available from Kohsei Co., Ltd; median particle diameter 1.9 mm$\Phi$; a carrier whose pore size distribution has sharp peaks in two regions of a macro-pore region (average pore size of 10 $\mu$m) and a meso-pore region (average pore size of 4 nm) was used (hereinafter, this carrier is referred to as a "binary porous silica carrier"). Separately, 3.09 g of boric acid and 7.32 g of diammonium hydrogenphosphate was dissolved in 22.2 g of distilled water in sequence, and 0.97 g of cesium nitrate as a metal source compound was added thereto, and the resultant was heated to 50°C while stirring, thereby obtaining a catalyst solution. The catalyst solution was added dropwise little by little onto 30 g of the binary porous silica carrier, which had been dried at 120°C for a day, and the resultant was left at room temperature for one hour, whereby the binary porous silica carrier was impregnated with the catalyst solution. The carrier impregnated with the catalyst solution was dried on an evaporating dish installed on a hot-water bath of 100°C to obtain a solid matter. The solid matter was dried at 120°C under airflow for 24 hours, and further calcined at 800°C in air atmosphere for 5 hours. Thus obtained calcined product was used as a catalyst. The obtained catalyst was a catalyst for glycerin dehydration in which boron phosphate and cesium as a metal element was supported on the binary porous silica carrier.

Examples C50 to C55 (Preparation of supported catalyst by an impregnation method)

Catalysts were prepared in the same manner as in Example C49, except that the used amounts of the binary porous silica carrier, boric acid, cesium nitrate, diammonium hydrogenphosphate and distilled water were changed as described in Table 15.

[Table 15]

| | Catalyst Preparing Method | Molar Ratio of Catalyst Component (Si : Molar Ratio of Carrier) | | | | | Amounts of Raw Materials (g) | | | | | |
| --- | --- | --- | --- | --- | --- | --- | --- | --- | --- | --- | --- | --- |
| | | M | P | B | Si | M/(P+B) | CsNO$_3$ | Boric Acid | Phosphate source Compound | Distilled Water | Carrier | |
| Example C44 | impregnation Method | 0.3 | 1.1 | 1.0 | 10 | 0.14 | 2.89 | 3.09 | 85% H$_3$PO$_4$ | 6.33 | 33.27 | Silica Carrier (Cariact Q50) | 30 |
| Example C45 | | 0.05 | 1.1 | 1.0 | 5 | 0.024 | 0.96 | 6.17 | | 12.66 | 26.94 | | 30 |
| Example C46 | | 0.1 | 1.1 | 1.0 | 5 | 0.048 | 1.93 | 6.17 | | 12.66 | 26.94 | | 30 |
| Example C47 | | 0.3 | 1.1 | 1.0 | 5 | 0.14 | 5.78 | 6.17 | | 12.66 | 26.94 | | 30 |
| Example C48 | | 0.5 | 1.1 | 1.0 | 5 | 0.24 | 9.63 | 6.17 | | 12.66 | 26.94 | | 30 |
| Example C49 | | 0.1 | 1.1 | 1.0 | 10 | 0.048 | 0.97 | 3.09 | (NH$_4$)$_2$ HPO$_4$ | 7.32 | 22.2 | Binary Porous Silica Carrier | 30 |
| Example C50 | | 0.3 | 1.1 | 1.0 | 10 | 0.14 | 2.92 | 3.09 | | 7.32 | 22.2 | | 30 |
| Example C51 | | 0.5 | 1.1 | 1.0 | 10 | 0.24 | 4.87 | 3.09 | | 7.32 | 22.2 | | 30 |
| Example C52 | | 0.7 | 1.1 | 1.0 | 10 | 0.33 | 6.81 | 3.09 | | 7.32 | 22.2 | | 30 |
| Example C53 | | 0.1 | 1.1 | 1.0 | 5 | 0.048 | 1.95 | 6.17 | | 14.63 | 22.2 | | 30 |
| Example C54 | | 0.3 | 1.1 | 1.0 | 5 | 0.14 | 5.84 | 6.17 | | 14.63 | 22.2 | | 30 |
| Example C55 | | 0.5 | 1.1 | 1.0 | 5 | 0.24 | 9.73 | 6.17 | | 14.63 | 22.2 | | 30 |

EP 2 886 193 A2

35

(3-2) Examples of acrolein production (initial performance)

Glycerin was dehydrated to produce acrolein according to the method described in the above section (1-2), using the catalyst obtained in each of the above Examples C1 to C55 and Comparative Examples C1 to C3. However, the effluent gas was collected for 30 minutes of from 4.5 hours to 5.0 hours from introducing the reaction gas into the reactor, and the effluent was subjected to the qualitative and quantitative analysis. Based on these analyses, the conversion rate of glycerin (GLY conversion rate), the selectivity of acrolein (ACR selectivity), and the selectivity of propionaldehyde (PALD selectivity) were calculated.

As shown in Table 16, comparing the results of Examples C1 to C 14 with the results of Comparative Examples C1 to C3, the catalyst comprising boron phosphate and a metal element wherein the molar ratio M/(P+B) of the metal element (M) to phosphorus (P) and boron (B) was more than 0.00005 and 0.5 or less, could enhance the selectivity of acrolein (ACR) and suppress the selectivity of propionaldehyde (PALD) at a low level. By using the catalyst comprising boron phosphate and a metal element, whose molar ratio M/(P+B) was adjusted in the prescribed range, it was found that acrolein could be produced in high yield while suppressing the production of propionaldehyde, especially, within byproducts.

[Table 16]

| | Metal Element (M) | Molar Ratio of Catalyst Component | | | | GLY Conv. Rate (%) | Selectivity (%) | |
|---|---|---|---|---|---|---|---|---|
| | | M | P | B | M/(P+B) | | ACR | PALD |
| Comp. Ex. C1 | - | - | 1.0 | 1.0 | 0 | 100 | 72.9 | 0.9 |
| Comp. Ex. C2 | Cs | 0.0001 | 1.0 | 1.0 | 0.000050 | 100 | 71.3 | 0.9 |
| Comp. Ex. C3 | Cs | 0.0001 | 1.1 | 1.0 | 0.000048 | 100 | 70.0 | 0.4 |
| Example C1 | Cs | 0.003 | 0.82 | 1.0 | 0.0016 | 100 | 76.0 | 0.5 |
| Example C2 | Cs | 0.01 | 1.0 | 1.0 | 0.0050 | 100 | 75.2 | 0.2 |
| Example C3 | Cs | 0.1 | 1.0 | 1.0 | 0.050 | 100 | 77.2 | 0.2 |
| Example C4 | Cs | 0.001 | 1.1 | 1.0 | 0.00048 | 100 | 73.9 | 0.4 |
| Example C5 | Cs | 0.005 | 1.1 | 1.0 | 0.0024 | 100 | 75.6 | 0.3 |
| Example C6 | Cs | 0.01 | 1.1 | 1.0 | 0.0048 | 100 | 76.6 | 0.2 |
| Example C7 | Cs | 0.05 | 1.1 | 1.0 | 0.024 | 100 | 74.9 | 0.2 |
| Example C8 | Cs | 0.1 | 1.1 | 1.0 | 0.048 | 100 | 76.9 | 0.3 |
| Example C9 | Cs | 0.5 | 1.1 | 1.0 | 0.24 | 100 | 76.9 | 0.3 |
| Example C10 | Cs | 0.01 | 1.2 | 1.0 | 0.0045 | 100 | 77.1 | 0.3 |
| Example C11 | Cs | 0.05 | 1.2 | 1.0 | 0.023 | 100 | 76.3 | 0.3 |
| Example C12 | Cs | 0.1 | 1.2 | 1.0 | 0.045 | 100 | 78.0 | 0.3 |
| Example C13 | Cs | 0.3 | 1.2 | 1.0 | 0.14 | 100 | 75.9 | 0.3 |
| Example C14 | Cs | 0.5 | 1.2 | 1.0 | 0.23 | 100 | 77.4 | 0.3 |

Table 17 shows the results of Examples C15 to C30. As shown in Table 17, the catalyst performance was not particularly affected by changing the catalyst preparing method from the condensation method to the kneading method; and the selectivity of acrolein (ACR) was kept high and the selectivity of propionaldehyde (PALD) was suppressed at a low level. In addition, even when the raw compound was changed from phosphoric acid to diammonium hydrogenphosphate and further from cesium nitrate to cesium carbonate, the catalyst performance was not significantly affected.

[Table 17]

| | Raw Compounds of Catalyst Component | | | Molar Ratio of Catalyst Component | | | | GLY Conv. Rate (%) | Selectivity (%) | |
|---|---|---|---|---|---|---|---|---|---|---|
| | M | P | B | M | P | B | M/(P+B) | | ACR | PALD |
| Example C15 | $CsNO_3$ | $(NH_4)_2$ $HPO_4$ | $H_3BO_3$ | 0.05 | 1.05 | 1.0 | 0.024 | 100 | 75.6 | 0.1 |
| Example C16 | | | | 0.1 | 1.05 | 1.0 | 0.049 | 100 | 75.3 | 0.1 |
| Example C17 | | | | 0.2 | 1.05 | 1.0 | 0.098 | 100 | 77.3 | 0.2 |
| Example C18 | | | | 0.3 | 1.05 | 1.0 | 0.15 | 100 | 76.2 | 0.2 |
| Example C19 | | | | 0.01 | 1.1 | 1.0 | 0.0048 | 100 | 77.0 | 0.1 |
| Example C20 | | | | 0.05 | 1.1 | 1.0 | 0.024 | 100 | 78.3 | 0.1 |
| Example C21 | | | | 0.1 | 1.1 | 1.0 | 0.048 | 100 | 77.4 | 0.1 |
| Example C22 | | | | 0.2 | 1.1 | 1.0 | 0.095 | 100 | 79.0 | 0.2 |
| Example C23 | | | | 0.3 | 1.1 | 1.0 | 0.14 | 100 | 80.7 | 0.2 |
| Example C24 | | | | 0.5 | 1.1 | 1.0 | 0.24 | 100 | 77.2 | 0.1 |
| Example C25 | $Cs_2CO_3$ | $(NH_4)_2$ $HPO_4$ | $H_3BO_3$ | 0.01 | 1.1 | 1.0 | 0.0048 | 100 | 76.0 | 0.1 |
| Example C26 | | | | 0.05 | 1.1 | 1.0 | 0.024 | 100 | 75.9 | 0.2 |
| Example C27 | | | | 0.1 | 1.1 | 1.0 | 0.048 | 100 | 78.8 | 0.2 |
| Example C28 | | | | 0.2 | 1.1 | 1.0 | 0.095 | 100 | 77.9 | 0.2 |
| Example C29 | | | | 0.3 | 1.1 | 1.0 | 0.14 | 100 | 78.4 | 0.2 |
| Example C30 | | | | 0.5 | 1.1 | 1.0 | 0.24 | 100 | 77.0 | 0.1 |

Table 18 shows the results of Examples C31 to C40. As shown in Table 18, the metal element was not limited to cesium, and also in the case where the alkali metal element such as lithium, sodium, potassium or rubidium, the alkaline-earth metal element such as magnesium, calcium or barium, the rare-earth metal element such as cerium, the copper group element such as silver, or the aluminum group element such as aluminum was used as the metal element, there was no particular problem about the catalyst performance as a catalyst for glycerin dehydration. In any case, the selectivity of propionaldehyde (PALD) was suppressed at a low level.

[Table 18]

| | Metal Element (M) | Molar Ratio of Catalyst Component | | | | GLY Conv. Rate (%) | Selectivity (%) | |
|---|---|---|---|---|---|---|---|---|
| | | M | P | B | M/(P+B) | | ACR | PALD |
| Example C31 | Li | 0.1 | 1.1 | 1.0 | 0.048 | 100 | 80.9 | 0.2 |

(continued)

| | Metal Element (M) | Molar Ratio of Catalyst Component | | | | GLY Conv. Rate (%) | Selectivity (%) | |
|---|---|---|---|---|---|---|---|---|
| | | M | P | B | M/(P+B) | | ACR | PALD |
| Example C32 | Na | 0.1 | 1.1 | 1.0 | 0.048 | 100 | 77.4 | 0.2 |
| Example C33 | Ag | 0.03 | 1.1 | 1.0 | 0.014 | 100 | 73.8 | 0.4 |
| Example C34 | Al | 0.004 | 1.1 | 1.0 | 0.0019 | 100 | 75.1 | 0.6 |
| Example C35 | K | 0.02 | 1.1 | 1.0 | 0.0095 | 100 | 84.3 | 0.2 |
| Example C36 | Rb | 0.02 | 1.1 | 1.0 | 0.0095 | 100 | 80.4 | 0.3 |
| Example C37 | Mg | 0.02 | 1.1 | 1.0 | 0.0095 | 100 | 78.2 | 0.2 |
| Example C38 | Ca | 0.02 | 1.1 | 1.0 | 0.0095 | 100 | 77.9 | 0.2 |
| Example C39 | Ba | 0.02 | 1.1 | 1.0 | 0.0095 | 100 | 78.8 | 0.2 |
| Example C40 | Ce | 0.02 | 1.1 | 1.0 | 0.0095 | 100 | 80.1 | 0.2 |

Table 19 shows the results of Examples C41 to C43. As shown in Table 19, the metal element was not limited to be only one kind to be used, and there was no particular problem about the catalyst performance as a catalyst for glycerin dehydration when two kinds of metal elements were used in combination.

[Table 19]

| | Metal Element ($M_1 + M_2$) | Molar Ratio of Catalyst Component | | | | | GLY Conv. Rate (%) | Selectivity (%) | |
|---|---|---|---|---|---|---|---|---|---|
| | | $M_1$ (Ce) | $M_2$ (Cs) | P | B | M/(P+B) | | ACR | PALD |
| Example C41 | | 0.01 | 0.1 | 1.1 | 1.0 | 0.0524 | 100 | 79.2 | 0.2 |
| Example C42 | Ce+Cs | 0.05 | 0.1 | 1.1 | 1.0 | 0.0714 | 100 | 80.7 | 0.2 |
| Example C43 | | 0.1 | 0.1 | 1.1 | 1.0 | 0.0952 | 100 | 82.0 | 0.1 |

Table 20 shows the results of Examples C44 to C55. As shown in Table 20, there was no particular problem about the catalyst performance as a catalyst for glycerin dehydration when the catalyst in which a catalyst component was supported on a carrier was used.

[Table 20]

| | Carrier | Molar Ratio of Catalyst Component (Si : Molar Ratio of Carrier) | | | | | GLY Conv. Rate (%) | Selectivity (%) | |
|---|---|---|---|---|---|---|---|---|---|
| | | M | P | B | Si | M/(P+B) | | ACR | PALD |
| Example C44 | | 0.3 | 1.1 | 1.0 | 10 | 0.14 | 100 | 73.7 | 0.5 |
| Example C45 | | 0.05 | 1.1 | 1.0 | 5 | 0.024 | 100 | 73.6 | 0.5 |
| Example C46 | Silica Carrier (Cariact Q50) | 0.1 | 1.1 | 1.0 | 5 | 0.048 | 100 | 73.7 | 0.3 |
| Example C47 | | 0.3 | 1.1 | 1.0 | 5 | 0.14 | 100 | 75.6 | 0.2 |
| Example C48 | | 0.5 | 1.1 | 1.0 | 5 | 0.24 | 100 | 75.3 | 0.3 |

(continued)

| | Carrier | Molar Ratio of Catalyst Component (Si : Molar Ratio of Carrier) | | | | | GLY Conv. Rate (%) | Selectivity (%) | |
|---|---|---|---|---|---|---|---|---|---|
| | | M | P | B | Si | M/(P+B) | | ACR | PALD |
| Example C49 | Binary Porous Silica Carrier | 0.1 | 1.1 | 1.0 | 10 | 0.048 | 100 | 73.9 | 0.3 |
| Example C50 | | 0.3 | 1.1 | 1.0 | 10 | 0.14 | 100 | 75.2 | 0.3 |
| Example C51 | | 0.5 | 1.1 | 1.0 | 10 | 0.24 | 100 | 75.0 | 0.2 |
| Example C52 | | 0.7 | 1.1 | 1.0 | 10 | 0.33 | 100 | 74.1 | 0.2 |
| Example C53 | | 0.1 | 1.1 | 1.0 | 5 | 0.048 | 100 | 78.8 | 0.2 |
| Example C54 | | 0.3 | 1.1 | 1.0 | 5 | 0.14 | 100 | 78.1 | 0.2 |
| Example C55 | | 0.5 | 1.1 | 1.0 | 5 | 0.24 | 100 | 77.7 | 0.2 |

(3-3) Examples of acrolein production (long-term performance; catalyst lifetime)

Example C56

Acrolein was produced according to the method described in the above section (3-2), using the catalyst obtained in the above Example C3 as the catalyst for glycerin dehydration. However, the reaction gas was allowed to flow in the reactor over 5 hours. During this time, for 30 minutes before the setup durations described in Table 21 or Table 22 from introducing the reaction gas into the reactor, effluent gas from the reactor was condensed in acetonitrile by cooling to recover and the effluent obtained at each of the setup durations was subjected to the qualitative and quantitative analysis. In Example C56, it was possible to produce acrolein for 48 hours without interruption.

Example C57

Acrolein was produced in the same manner as in Example C56, except using the catalyst obtained in the above Example C14 as the catalyst for glycerin dehydration. In Example C57, it was possible to produce acrolein for 48 hours without interruption.

Example C58

Acrolein was produced in the same manner as in Example C56, except using the catalyst obtained in the above Example C8 as the catalyst for glycerin dehydration. In Example C58, it was possible to produce acrolein for 80 hours without interruption.

Comparative Example C4

Acrolein was produced in the same manner as in Example C56, except using the catalyst obtained in the above Comparative Example C1 as the catalyst for glycerin dehydration. In Comparative Example C4, the pressure loss gradually increased as the reaction time goes by, and the production of acrolein became impossible after 20 hours passage of the reaction time.

Comparison of results of Examples C56 to C58 and Comparative Example C4

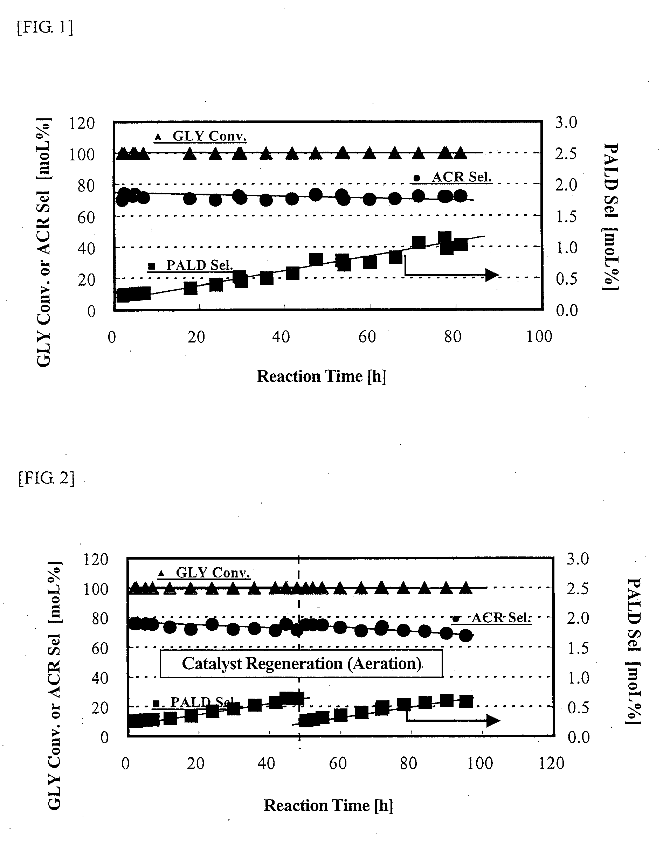

The results of Examples C56 to C58 and Comparative Example C4 are shown in Tables 21, 22 and Figure 1. Comparing the results of Examples C56 to C58 with the results of Comparative Example C4, the catalyst comprising boron phosphate and a metal element, whose molar ratio M/(P+B) was adjusted in the prescribed range, lengthened the reaction continuable time more than twice than the catalyst comprising boron phosphate but not comprising a metal element. Particularly, in Example C58, it was possible to continue the reaction over 80 hours. In Examples C56 and C57, though the reaction was stopped at 48 hours duration, it was possible to continue the reaction after that. In any case, decrease in the selectivity of acrolein (ACR) and increase in the selectivity of propionaldehyde (PALD) with time were observed.

[Table 21]

| | Molar Ratio of Cat. Comp. | | | Reaction Time (h) | GLY Conv. Rate (%) | Selectivity (%) | |
|---|---|---|---|---|---|---|---|
| | M | P | B | | | ACR | PALD |
| Comparative Example C4 | - | 1.0 | 1.0 | 1 | 100 | 71.1 | 0.8 |
| | | | | 5 | 100 | 69.2 | 0.9 |
| | | | | 7 | 100 | 68.8 | 1.0 |
| | | | | 12 | 100 | 72.8 | 0.9 |
| | | | | 18 | 100 | 72.3 | 1.0 |
| | | | | Reaction was stopped at 20 hrs. due to pressure loss increase | | | |
| Example C56 | 0.1 | 1.0 | 1.0 | 2 | 100 | 70.7 | 0.1 |
| | | | | 2.5 | 100 | 74.8 | 0.2 |
| | | | | 4.5 | 100 | 73.9 | 0.2 |
| | | | | 5 | 100 | 74.7 | 0.2 |
| | | | | 7 | 100 | 74.3 | 0.2 |
| | | | | 24 | 100 | 70.7 | 0.4 |
| | | | | 29.5 | 100 | 71.6 | 0.5 |
| | | | | 30 | 100 | 73.8 | 0.5 |
| | | | | 47.5 | 100 | 71.1 | 0.7 |
| | | | | 48 | 100 | 70.9 | 0.8 |
| Example C57 | 0.5 | 1.2 | 1.0 | 2 | 100 | 80.1 | 0.2 |
| | | | | 2.5 | 100 | 74.8 | 0.2 |
| | | | | 5 | 100 | 72.3 | 0.3 |
| | | | | 7 | 100 | 73.1 | 0.3 |
| | | | | 24 | 100 | 72.1 | 0.5 |
| | | | | 27.5 | 100 | 74.5 | 0.5 |
| | | | | 29.5 | 100 | 71.3 | 0.5 |
| | | | | 30 | 100 | 70.4 | 0.5 |
| | | | | 47.5 | 100 | 73.3 | 0.7 |
| | | | | 48 | 100 | 72.0 | 0.8 |

[Table 22]

| | Molar Ratio of Cat. Comp. | | | Reaction Time (h) | GLY Conv. Rate (%) | Selectivity (%) | |
|---|---|---|---|---|---|---|---|
| | M | P | B | | | ACR | PALD |
| Example C58 | 0.1 | 1.1 | 1.0 | 2 | 100 | 70.2 | 0.2 |
| | | | | 2.5 | 100 | 74.1 | 0.2 |
| | | | | 4.5 | 100 | 72.5 | 0.2 |
| | | | | 5 | 100 | 73.7 | 0.3 |
| | | | | 7 | 100 | 71.6 | 0.3 |
| | | | | 18 | 100 | 71.0 | 0.3 |
| | | | | 24 | 100 | 70.1 | 0.4 |
| | | | | 29.5 | 100 | 72.6 | 0.5 |
| | | | | 30 | 100 | 71.2 | 0.5 |
| | | | | 36 | 100 | 70.0 | 0.5 |
| | | | | 42 | 100 | 70.8 | 0.6 |
| | | | | 47.5 | 100 | 73.4 | 0.8 |
| | | | | 53.5 | 100 | 72.9 | 0.8 |
| | | | | 54 | 100 | 70.4 | 0.7 |
| | | | | 60 | 100 | 70.5 | 0.8 |
| | | | | 66 | 100 | 70.8 | 0.8 |
| | | | | 71.5 | 100 | 72.5 | 1.1 |
| | | | | 77.5 | 100 | 72.2 | 1.1 |
| | | | | 78 | 100 | 72.2 | 1.0 |
| | | | | 81.25 | 100 | 72.3 | 1.0 |

(3-4) Examples of acrolein production including a catalyst regeneration treatment (long-term performance)

Example C59

Acrolein was produced according to the method described in the above section (3-2), using the catalyst obtained in the above Example C8 as the catalyst for glycerin dehydration. After feeding the reaction gas into the reactor for 48 hours, the production of acrolein was suspended, and the reaction gas or the production remained in the reactor was discharged by introducing nitrogen gas into the fixed-bed reactor at the rate of 62 mL/min for 30 minutes. Thereafter, air was allowed to flow in the reactor at the rate of 62 mL/min for 24 hours, thereby conducting a catalyst regeneration treatment of removing carbonaceous matters deposited on the catalyst. The temperature of the molten salt bath in which the fixed-bed reactor was immersed was kept at 360°C, which temperature was the same at the acrolein production. Subsequently, production of acrolein was conducted again for 48 hours without interruption, according to the above acrolein production.

The obtained results are shown in Table 23 and Figure 2. Till 48 hours of the reaction time, the selectivity of acrolein (ACR) decreased and the selectivity of propionaldehyde (PALD) increased with time. However, when the catalyst regeneration treatment was conducted, and then, the production of acrolein was conducted subsequently, it was found that both the selectivity of acrolein (ACR) and the selectivity of propionaldehyde (PALD) were resurged to the same level as the initial stage.

[Table 23]

| | Molar Ratio of Cat. Comp. | | | Integrating Reaction Time (h) | GLY Conv. Rate (%) | Selectivity (%) | |
|---|---|---|---|---|---|---|---|
| | M | P | B | | | ACR | PALD |
| | | | | 2 | 100 | 75.8 | 0.3 |
| | | | | 2.5 | 100 | 75.8 | 0.3 |
| | | | | 5 | 100 | 75.6 | 0.3 |
| | | | | 7 | 100 | 75.2 | 0.3 |
| | | | | 12 | 100 | 73.3 | 0.3 |
| | | | | 18 | 100 | 71.9 | 0.3 |
| | | | | 24 | 100 | 75.3 | 0.4 |
| | | | | 30 | 100 | 71.9 | 0.5 |
| | | | | 36 | 100 | 72.5 | 0.5 |
| | | | | 42 | 100 | 71.0 | 0.6 |
| | | | | 45 | 100 | 75.4 | 0.6 |
| Example C59 | 0.1 | 1.1 | 1.0 | 48 | 100 | 71.5 | 0.6 |
| | | | | Catalyst regeneration | | | |
| | | | | 50.5 | 100 | 75.1 | 0.3 |
| | | | | 52.5 | 100 | 75.2 | 0.3 |
| | | | | 55 | 100 | 74.8 | 0.3 |
| | | | | 60 | 100 | 73.1 | 0.4 |
| | | | | 66 | 100 | 70.8 | 0.4 |
| | | | | 71.5 | 100 | 72.1 | 0.5 |
| | | | | 72 | 100 | 73.6 | 0.5 |
| | | | | 78 | 100 | 71.1 | 0.5 |
| | | | | 84 | 100 | 70.6 | 0.6 |
| | | | | 90 | 100 | 69.2 | 0.6 |
| | | | | 95.5 | 100 | 67.5 | 0.6 |

(3-5) Example of acrylic acid production

Acrylic acid was produced from glycerin according to the producing process described in the above section (1-4) except the following points.

(i) First reaction step

A catalyst obtained in Example C3 was used as a catalyst used for the first reaction (a catalyst for the first reaction). The weight of the recovered reaction product was 939 g, which corresponded to 99 mass% of the supplied raw material. The obtained reaction product contained 38 mass% of acrolein, 0.1 mass% of propionaldehyde, 9.5 mass% of 1-hydroxyacetone, 46 mass% of water and 6 mass% of heavy components.

The reaction product obtained in the step (i) was supplied at 0.12 kg/h to a thin film distiller, which was operated in the conditions of normal pressure, 85°C of wall temperature on which a liquid film was formed, and 300 rpm of blade rotation speed. A distillate containing 43 mass% of acrolein, 0.1 mass% of propionaldehyde, 6 mass% of 1-hydroxyacetone and 51 mass% of water was obtained at 0.11 kg/h from the top of the distiller.

(iii) Second reaction step

As the propionaldehyde-containing gas, a mixed gas consisting of 6.5 volume% of acrolein, 0.02 volume% of propionaldehyde, 0.62 volume% of 1-hydroxyacetone, 6 volume% of oxygen, 13 volume% of water and 74 volume% of nitrogen was used. The weight of the recovered reaction product was 625 g, which corresponded to 95 mass% of the supplied raw material. The reaction product contained 62 mass% of acrylic acid, 0.01 mass% of propionic acid, 1 mass% of formic acid, 3 mass% of acetic acid and 34 mass% of water.

(iv) Second purification step

The obtained crude acrylic acid contained 88.1 mass% of acrylic acid, 0.01 mass% of propionic acid, 2.3 mass% of acetic acid, 0.04 mass% of formic acid and 9.5 mass% of water. In the crystallization operation, the mother liquid was cooled to a temperature range of -5.8°C to room temperature (about 15°C) to form a crystal at the first crystallization, and the mother liquid was cooled to a temperature range of 4.8°C to room temperature (about 15°C) to form a crystal at the second crystallization. The finally obtained acrylic acid had a purity of 99.9 mass% or higher, and the content of propionic acid was below a detection limit (1 ppm), as shown in Table 24.

[Table 24]

|  | Mother liquid | First crystallization | Second crystallization |
|---|---|---|---|
| Acrylic acid (mass%) | 88.1 | 98.8 | 99.9 |
| Propionic acid (mass%) | 0.01 | N.D. | N.D. |
| Acetic acid (mass%) | 2.3 | 0.3 | 0.02 |
| Water (mass%) | 9.5 | 0.9 | 0.08 |
| Temperature (°C) | - | -5.8 | 4.8 |

N.D. means a value below the detection limit (1 ppm by mass).

(3-6) Example of production of a water-absorbent resin

A water-absorbent resin was produced from acrylic acid according to the producing process described in the above section (1-5). The obtained acrylic acid had the comparable polymerization property to acrylic acid obtained by the process of producing acrylic acid from propylene, and the obtained hydrophilic resin was free from odor and had the same physical properties as a conventional one.

(4) Catalyst comprising a rare-earth metal phosphate and a metal element

(4-1) Preparation of catalyst

Example D1 (Preparation of catalyst by a kneading method)

69.55 g of finely-pulverized neodymium oxide (available from Kanto Chemical Co., Inc.), 55.04 g of diammonium hydrogenphosphate, and 1.61 g of cesium nitrate (available from Chemetall GmbH) were weighed and fed to a mortar in sequence, and then, mixed for 30 minutes while being pulverized by a pestle. The obtained mixture was dried under air atmosphere at 120°C for 24 hours. Thus obtained dried product was placed in air atmosphere at 450°C for 10 hours (a pre-heat treatment) for the purpose of separating and decomposing ammonium components which is assumed to exist in the dried product, followed by calcinating under air atmosphere at 1000°C for 5 hours to give a calcinated product. The calcinated product was sieved by using sieves with opening apertures of 0.7 mm or 2.0 mm, thereby obtaining a classified calcinated product with the size of 0.7 mm to 2.0 mm as a catalyst. The obtained catalyst was a catalyst for glycerin dehydration comprising neodymium phosphate and cesium as a metal element.

Examples D2 to D6 and Comparative Examples D1, D2 (Preparation of catalyst by a kneading method)

Catalysts were prepared in the same manner as in Example D1, except that the used amounts of neodymium oxide, diammonium hydrogenphosphate and cesium nitrate were changed as described in Table 25.

Examples D7, D8 (Preparation of catalyst by a kneading method)

Catalysts were prepared in the same manner as in Example D1, except that the used amounts of neodymium oxide and diammonium hydrogenphosphate were changed as described in Table 25 and potassium nitrate was used by the amount described in Table 25 in place of cesium nitrate.

[Table 25]

| | Catalyst Preparing Method | Molar Ratio of Catalyst Component | | | | Amounts of Raw Materials (g) | | | |
|---|---|---|---|---|---|---|---|---|---|
| | | M | P | R(Nd) | M/(P+R) | Metal Source Compound | | $Nd_2O_3$ | $(NH_4)_2HPO_4$ |
| Comp. Ex. D1 | Kneading Method | 0 | 1.0 | 1.0 | 0 | - | - | 70.37 | 55.68 |
| Comp. Ex. D2 | | 0.0001 | 1.1 | 1.0 | 0.000048 | $CsNO_3$ | 0.01 | 68.33 | 59.48 |
| Example D1 | | 0.02 | 1.0 | 1.0 | 0.01 | | 1.61 | 69.55 | 55.04 |
| Example D2 | | 0.001 | 1.1 | 1.0 | 0.00048 | | 0.08 | 68.30 | 59.45 |
| Example D3 | | 0.01 | 1.1 | 1.0 | 0.0048 | | 0.79 | 67.95 | 59.15 |
| Example D4 | | 0.02 | 1.1 | 1.0 | 0.0095 | | 1.56 | 67.57 | 58.81 |
| Example D5 | | 0.05 | 1.1 | 1.0 | 0.24 | | 3.85 | 66.44 | 57.83 |
| Example D6 | | 0.1 | 1.1 | 1.0 | 0.048 | | 7.49 | 64.64 | 56.27 |
| Example D7 | | 0.01 | 1.1 | 1.0 | 0.0048 | $KNO_3$ | 0.82 | 68.08 | 59.26 |
| Example D8 | | 0.02 | 1.1 | 1.0 | 0.0095 | | 1.63 | 67.82 | 59.04 |

Example D9 (Preparation of catalyst by a sol-gel method)

Into the aqueous solution obtained by adding 1800 g of distilled water to 455.86 g of $Nd(NO_3)_3$ aqueous solution (available from Shin-Etsu Chemical Co., Ltd.; concentration in terms of $Nd_2O_3$: 221 g/kg), 2.38 g of cesium nitrate (available from Chemetall GmbH) was added and well-stirred to give a uniform solution. Into the obtained solution under stirring, 185.43 g of 28 wt% ammonia water was added dropwise at a constant rate over 3 hours using a pump for high-performance liquid chromatography ("L7110" available from Hitachi, Ltd.). The solution after the dropwise addition was aged by being left for 15 hours under stirring to obtain a solution containing neodymium hydroxide and cesium. Into this solution under stirring, 77.32 g of phosphoric acid aqueous solution ($H_3PO_4$ concentration: 85 mass%) was added dropwise at a constant rate over 3 hours using a pump for high-performance liquid chromatography. The solution after the dropwise addition was left for 15 hours under stirring to obtain a sol- or gel-like material containing phosphate, neodymium and cesium. The sol- or gel-like material was dewatered under the conditions of 0.005 MPa and 60°C, followed by drying under air atmosphere at 120°C for 10 hours. Thus obtained dried product was placed in nitrogen atmosphere at 450°C for 10 hours (a pre-heat treatment) for the purpose of separating and decomposing ammonium components which is assumed to exist in the dried product, followed by calcinating under air atmosphere at 1000°C for 5 hours to give a calcinated product. The calcinated product was pulverized and sieved by using sieves with opening apertures of 0.7 mm or 2.0 mm, thereby obtaining a classified calcinated product with the size of 0.7 mm to 2.0 mm as a catalyst. The obtained catalyst was a catalyst for glycerin dehydration comprising neodymium phosphate and cesium as a metal element.

Examples D10, D11 and Comparative Examples D3 (Preparation of catalyst by a sol-gel method)

Catalysts were prepared in the same manner as in Example D9, except that the used amounts of $Nd(NO_3)_3$ aqueous solution, cesium nitrate, ammonia water and phosphoric acid aqueous solution were changed as described in Table 26.

[Table 26]

| | Catalyst Preparing Method | Molar Ratio of Catalyst Component | | | | Amounts of Raw Materials (g) | | | |
|---|---|---|---|---|---|---|---|---|---|
| | | M(Cs) | P | R(Nd) | M/(P+R) | $CsNO_3$ | $Nd(NO_3)_3$ aq. solution | $H_3PO_4$ aq. solution | Ammonia Water |
| Comp. Ex. D3 | Sol-gel Method | 0 | 1.1 | 1.0 | 0 | - | 461.14 | 78.22 | 187.58 |
| Example D9 | | 0.02 | 1.1 | 1.0 | 0.0095 | 2.38 | 455.86 | 77.32 | 185.43 |
| Example D10 | | 0.05 | 1.1 | 1.0 | 0.24 | 5.84 | 448.16 | 76.02 | 182.30 |
| Example D11 | | 0.1 | 1.1 | 1.0 | 0.048 | 11.36 | 435.89 | 73.93 | 177.31 |

Example D12 (Preparation of catalyst by a sol-gel method)

Catalyst was prepared in the same manner as in Example D9, except using the aqueous solution obtained by adding 826.5 g of distilled water to 197.8 g of yttrium nitrate (available from Kishida Chemical Co., Ltd.) in place of the aqueous solution obtained by adding 1800 g of distilled water to 455.86 g of $Nd(NO_3)_3$ aqueous solution, and changing the used amounts of cesium nitrate, ammonia water and phosphoric acid aqueous solution as described in Table 27. Calcination was conducted at 900°C. The obtained catalyst was a catalyst for glycerin dehydration comprising yttrium phosphate and cesium as a metal element.

Examples D13, D 14 (Preparation of catalyst by a sol-gel method)

Catalysts were prepared in the same manner as in Example D12, except that the used amounts of cesium nitrate, yttrium nitrate, distilled water, ammonia water and phosphoric acid aqueous solution were changed as described in Table 27. In Example D13, calcination was conducted at 1100°C, and in Example D14, calcination was conducted at 1000°C.

[Table 27]

| | Catalyst Preparing Method | Molar Ratio of Catalyst Component | | | | Amounts of Raw Materials (g) | | | | |
|---|---|---|---|---|---|---|---|---|---|---|
| | | M(Cs) | P | R(Y) | M/(P+R) | $CsNO_3$ | $Y(NO_3)_3 .6H_2O$ | Distilled Water | $H_3PO_4$ aq. solution | Ammonia Water |
| Example D 12 | Sol-gel Method | 0.02 | 1.1 | 1.0 | 0.0095 | 2.01 | 197.8 | 826.5 | 65.4 | 156.9 |
| Example D 13 | | 0.05 | 1.1 | 1.0 | 0.024 | 4.92 | 193.6 | 836.4 | 64.0 | 153.6 |
| Example D 14 | | 0.1 | 1.1 | 1.0 | 0.048 | 9.51 | 187.0 | 851.9 | 61.8 | 148.3 |

Examples D 15 to D 17 (Preparation of catalyst by a sol-gel method)

Catalyst were prepared in the same manner as in Example D12, except that cerium nitrate was used by the amounts of described in Table 28 in place of yttrium nitrate and the used amounts of cesium nitrate, distilled water, ammonia water and phosphoric acid aqueous solution were changed as described in Table 28. In Examples D15 and D16, calcination was conducted at 1000°C, and in Example D17, calcination was conducted at 1100°C. The obtained catalysts were catalysts for glycerin dehydration comprising cerium phosphate and cesium as a metal element.

[Table 28]

| | Catalyst Preparing Method | Molar Ratio of Catalyst Component | | | | Amounts of Raw Materials (g) | | | | |
|---|---|---|---|---|---|---|---|---|---|---|
| | | M(Cs) | P | R(Ce) | M/(P+R) | $CsNO_3$ | $Ce(NO_3)_3 . 6H_2O$ | Distilled Water | $H_3PO_4$ aq. solution | Ammonia Water |
| Example D15 | | 0.02 | 1.1 | 1.0 | 0.0095 | 1.59 | 177.4 | 931.4 | 51.8 | 124.1 |
| Example D16 | Sol-gel Method | 0.05 | 1.1 | 1.0 | 0.024 | 3.91 | 174.4 | 937.5 | 50.9 | 122.0 |
| Example D17 | | 0.1 | 1.1 | 1.0 | 0.048 | 7.61 | 169.6 | 947.2 | 49.5 | 118.7 |

(4-2) Examples of acrolein production (initial performance)

Glycerin was dehydrated to produce acrolein according to the method described in the above section (1-2), using the catalyst obtained in each of the above Examples D1 to D17 and Comparative Examples D1 to D3, and the conversion rate of glycerin (GLY conversion rate), the selectivity of acrolein (ACR selectivity), and the selectivity of propionaldehyde (PALD selectivity) were calculated. The results where the catalysts prepared by the kneading method were employed are shown in Table 29 and the results where the catalysts prepared by the sol-gel method were employed are shown in Table 30.

In the case of using the catalyst prepared by the kneading method (Table 29), in Comparative Example D1 where the catalyst comprising neodymium phosphate but not comprising a metal element was used and Comparative Example D2 where the catalyst comprising neodymium phosphate and a metal element (cesium) and having the molar ratio M/(P+R), molar ratio of the metal element (M) to phosphorus (P) and the rare-earth metal (R), of 0.000048 was used, the selectivity of acrolein (ACR) was around 65% and the selectivity of propionaldehyde (PALD) was 0.38% or higher. Meanwhile, in Examples D1 to D8 where the catalysts comprising neodymium phosphate and a metal element (cesium or potassium) and having the molar ratio M/(P+R) of more than 0.00005 and 0.5 or less were used, the selectivity of acrolein (ACR) rose to 70% or higher and the selectivity of propionaldehyde (PALD) decreased to in the range of 0.12% to 0.24%, which values were smaller more than about one-third of those in Comparative Examples D1 and D2.

[Table 29]

| | Metal Element (M) | Molar Ratio of Catalyst Component | | | | GLY Conv. Rate (%) | Selectivity (%) | |
|---|---|---|---|---|---|---|---|---|
| | | M | P | R(Nd) | M/(P+R) | | ACR | PALD |
| Comp. Ex. D1 | - | 0 | 1.0 | 1.0 | 0 | 100 | 65.9 | 0.62 |
| Comp. Ex. D2 | Cs | 0.0001 | 1.1 | 1.0 | 0.000048 | 100 | 65.3 | 0.38 |
| Example D1 | Cs | 0.02 | 1.0 | 1.0 | 0.01 | 100 | 74.6 | 0.22 |
| Example D2 | Cs | 0.001 | 1.1 | 1.0 | 0.00048 | 100 | 70.0 | 0.18 |
| Example D3 | Cs | 0.01 | 1.1 | 1.0 | 0.0048 | 100 | 75.2 | 0.14 |
| Example D4 | Cs | 0.02 | 1.1 | 1.0 | 0.0095 | 100 | 75.3 | 0.15 |
| Example D5 | Cs | 0.05 | 1.1 | 1.0 | 0.24 | 100 | 75.3 | 0.13 |
| Example D6 | Cs | 0.1 | 1.1 | 1.0 | 0.048 | 100 | 72.4 | 0.12 |
| Example D7 | K | 0.01 | 1.1 | 1.0 | 0.0048 | 100 | 73.8 | 0.24 |
| Example D8 | K | 0.02 | 1.1 | 1.0 | 0.0095 | 100 | 75.2 | 0.17 |

In the case of using the catalyst prepared by the sol-gel method (Table 30), in Comparative Example D3 where the catalyst comprising neodymium phosphate but not comprising a metal element was used, the selectivity of acrolein (ACR) was 68.9% and the selectivity of propionaldehyde (PALD) was 1.14%. Meanwhile, in Examples D9 to D11 where the catalysts comprising neodymium phosphate and a metal element (cesium) and having the molar ratio M/(P+R) of more than 0.00005 and 0.5 or less were used, the selectivity of acrolein (ACR) rose to 77% or higher and the selectivity of propionaldehyde (PALD) decreased to in the range of 0.24% to 0.37%, which values were smaller more than about two-third of that in Comparative Example D3. In each of Examples D12 to D17 where yttrium phosphate or cerium phosphate in place of neodymium phosphate was used as the rare-earth metal phosphate, the selectivity of acrolein (ACR) was nearly equal to or surpassed that in Comparative Example D3 and the selectivity of propionaldehyde (PALD) decreased. Particularly in Examples D13 and D16, the selectivity of acrolein (ACR) increased and the selectivity of propionaldehyde (PALD) decreased, compared with the case where neodymium phosphate was used as the rare-earth metal phosphate (Examples D9 to D11), and the excellent catalyst performance were demonstrated.

[Table 30]

| | Rare-earth Metal (R) | Molar Ratio of Catalyst Component | | | | GLY Conv. Rate (%) | Selectivity (%) | |
|---|---|---|---|---|---|---|---|---|
| | | M(Cs) | P | R | M/(P+R) | | ACR | PALD |
| Comp. Ex. D3 | - | 0 | 1.1 | 1.0 | 0 | 100 | 68.9 | 1.14 |

(continued)

| | Rare-earth Metal (R) | Molar Ratio of Catalyst Component | | | | GLY Conv. Rate (%) | Selectivity (%) | |
|---|---|---|---|---|---|---|---|---|
| | | M(Cs) | P | R | M/(P+R) | | ACR | PALD |
| Example D9 | Nd | 0.02 | 1.1 | 1.0 | 0.0095 | 100 | 77.2 | 0.37 |
| Example D10 | Nd | 0.05 | 1.1 | 1.0 | 0.24 | 100 | 78.0 | 0.24 |
| Example D11 | Nd | 0.1 | 1.1 | 1.0 | 0.048 | 100 | 77.1 | 0.29 |
| Example D12 | Y | 0.02 | 1.1 | 1.0 | 0.0095 | 100 | 69.2 | 0.37 |
| Example D13 | Y | 0.05 | 1.1 | 1.0 | 0.24 | 100 | 78.3 | 0.11 |
| Example D14 | Y | 0.1 | 1.1 | 1.0 | 0.048 | 100 | 67.8 | 0.28 |
| Example D15 | Ce | 0.02 | 1.1 | 1.0 | 0.0095 | 100 | 69.4 | 0.59 |
| Example D16 | Ce | 0.05 | 1.1 | 1.0 | 0.24 | 100 | 78.1 | 0.21 |
| Example D17 | Ce | 0.1 | 1.1 | 1.0 | 0.048 | 100 | 74.2 | 0.27 |

(4-3) Example of acrylic acid production

Acrylic acid was produced from glycerin according to the producing process described in the above section (1-4) except the following points.

(i) First reaction step

A catalyst obtained in Example D5 was used as a catalyst used for the first reaction (a catalyst for the first reaction), and the glycerin-containing gas was supplied for 24 hours. The weight of the recovered reaction product was 1026 g, which corresponded to 99 mass% of the supplied raw material. The obtained reaction product contained 39.0 mass% of acrolein, 0.1 mass% of propionaldehyde, 11.0 mass% of 1-hydroxyacetone, 45 mass% of water and 4.9 mass% of heavy components.

(ii) First purification step

The reaction product obtained in the step (i) was supplied at 0.48 kg/h to the fifth stage of an Oldershow continuous distillation column having 10 stages under the conditions of a refluxing ratio of 2 and the bottom temperature of 94°C. A distillate containing 98 mass% of acrolein, 0.1 mass% of propionaldehyde and 2 mass% of water was obtained at 0.16 kg/h from the top of the distiller.

(iii) Second reaction step

As the propionaldehyde-containing gas, a mixed gas consisting of 6.5 volume% of acrolein, 0.01 volume% of propionaldehyde, 6 volume% of oxygen, 13 volume% of water and 74.5 volume% of nitrogen was introduced at a space velocity (GHSV) of 1600 hr$^{-1}$. The weight of the recovered reaction product was 505 g, which corresponded to 99 mass% of the supplied raw material. The reaction product contained 67.8 mass% of acrylic acid, 0.10 mass% of propionic acid, 0.5 mass% of formic acid, 0.93 mass% of acetic acid and 30.7 mass% of water.

(iv) Second purification step

The obtained crude acrylic acid contained 89.2 mass% of acrylic acid, 0.12 mass% of propionic acid, 0.85 mass% of acetic acid, 0.05 mass% of formic acid and 9.74 mass% of water. In the crystallization operation, the mother liquid was cooled to a temperature range of -5.8°C to room temperature (about 15°C) to form a crystal at the first crystallization, and the mother liquid was cooled to a temperature range of 4.8°C to room temperature (about 15°C) to form a crystal at the second crystallization. The finally obtained acrylic acid had a purity of 99.9 mass% or higher, and the content of propionic acid was below a detection limit (1 ppm), as shown in Table 31.

[Table 31]

|  | Mother liquid | First crystallization | Second crystallization |
|---|---|---|---|
| Acrylic acid (mass%) | 89.2 | 98.8 | 99.9 |
| Propionic acid (mass%) | 0.12 | N.D. | N.D. |
| Acetic acid (mass%) | 0.85 | 0.2 | 0.02 |
| Water (mass%) | 9.74 | 0.9 | 0.10 |
| Temperature (°C) | - | -5.8 | 4.8 |
| N.D. means a value below the detection limit (1 ppm by mass). | | | |

(4-4) Example of production of a water-absorbent resin

A water-absorbent resin was produced from acrylic acid according to the producing process described in the above section (1-5). The obtained acrylic acid had the comparable polymerization property to acrylic acid obtained by the process of producing acrylic acid from propylene, and the obtained hydrophilic resin was free from odor and had the same physical properties as a conventional one.

The present invention enables efficient production of acrolein from glycerin. Therefore, the present invention can enormously contribute to the spread of biodiesel and global warming countermeasure as am important technology for promoting the effective utilization of glycerin, which is a by-product of the production of biodiesel.

**Claims**

1. A catalyst for glycerin dehydration,
   comprising a rare-earth metal phosphate,
   wherein a molar ratio P/R of phosphorus (P) to a rare-earth metal (R) is more than 1.0 and 2.0 or less.

2. A catalyst for glycerin dehydration,
   comprising a combination of a rare-earth metal phosphate and a metal element other than a rare-earth metal,
   wherein a molar ratio M/(P+R) of a metal element (M) to phosphorus (P) and a rare-earth metal (R) is more than 0.00005 and 0.5 or less.

3. The catalyst for glycerin dehydration according to claim 2, wherein the metal element is at least one element selected from the group consisting of alkali metal elements, alkaline-earth metal elements, iron group elements, platinum group elements, copper group elements, and aluminum group elements.

4. The catalyst for glycerin dehydration according to any one of claims 1 to 3, wherein the rare-earth metal is at least one element selected from the group consisting of yttrium, lanthanum, cerium, praseodymium, neodymium, and gadolinium.

5. A process for producing acrolein, comprising the step of dehydrating glycerin in the presence of the catalyst according to any one of claims 1 to 4, to produce acrolein.

6. The process for producing acrolein according to claim 5, wherein glycerin is dehydrated by gas-phase reaction of bringing a reaction gas containing glycerin into contact with the catalyst, to produce acrolein.

7. A process for producing acrylic acid, comprising the step of oxidizing acrolein produced by the process according to claim 5 or 6, to produce acrylic acid.

8. A process for producing a hydrophilic resin, comprising the step of polymerizing a monomeric component including acrylic acid produced by the process according to claim 7.

9. The process for producing a hydrophilic resin according to claim 8, wherein the hydrophilic resin is a water-absorbent resin.

[FIG. 1]

[FIG. 2]

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007119528 A
- JP 2008307521 A
- JP 2009263284 A
- JP 2009274982 A
- JP 2008115103 A
- JP 9227445 A
- JP 2002519402 A
- US 6107358 A
- US 6174978 B
- US 6241928 B
- US 6867269 B
- US 6906159 B
- US 7091253 B
- WO 01038402 A
- WO 2006034806 A
- US 4625001 A
- US 4873299 A
- US 4286082 A
- US 4973632 A
- US 4985518 A
- US 5124416 A
- US 5250640 A
- US 5264495 A
- US 5145906 A
- US 5380808 A
- EP 0811636 A
- EP 0955086 A
- EP 0922717 A
- US 4093776 A
- US 4367323 A
- US 4446261 A
- US 4683274 A
- US 5244735 A